# EUROPEAN PATENT SPECIFICATION

(11) **EP 2 513 336 B1**
(45) Date of publication and mention of the grant of the patent: **02.03.2016**
(21) Application number: 10842460.7
(22) Date of filing: 08.12.2010
(51) Int. Cl.: C12Q 1/68, C12P 19/34, G01N 33/574

(54) **METHOD FOR CHARACTERIZING HOST IMMUNE FUNCTION BY EX VIVO INDUCTION OF OFFENSIVE AND DEFENSIVE IMMUNE MARKERS**
VERFAHREN ZUR BESTIMMUNG DER IMMUNFUNKTION EINES WIRTES DURCH EX-VIVO-EINFÜHRUNG OFFENSIVER UND DEFENSIVER IMMUNMARKER
PROCÉDÉ POUR CARACTÉRISER LA FONCTION IMMUNITAIRE DE L'HÔTE PAR INDUCTION EX VIVO DE MARQUEURS IMMUNITAIRES OFFENSIFS ET DÉFENSIFS

(30) Priority: 16.12.2009 US 287114 P
(43) Date of publication of application: 24.10.2012
(73) Proprietor: Hitachi Chemical Co., Ltd., Chiyoda-ku Tokyo 100-6606 (JP); Hitachi Chemical Co. America, Ltd., Cupertino, CA 95014 (US)
(72) Inventor: MITSUHASHI, Masato, Irvine, CA 92614 (US)
(74) Representative: Vossius & Partner Patentanwälte Rechtsanwälte mbB
(86) International application number: PCT/US2010/059552
(87) International publication number: WO 2011/084333

(56) References cited:
- US-A1- 2007 122 877
- US-A1- 2009 111 128
- US-A1- 2009 136 447
- US-A1- 2009 203 064
- MITSUHASHI ET AL: "Ex vivo simulation of leukocyte function: Stimulation of specific subset of leukocytes in whole blood followed by the measurement of function-associated mRNAs", JOURNAL OF IMMUNOLOGICAL METHODS, ELSEVIER SCIENCE PUBLISHERS B.V.,AMSTERDAM, NL, vol. 363, no. 1, 15 December 2010 (2010-12-15), pages 95-100, XP027536029, ISSN: 0022-1759 [retrieved on 2010-10-15]
- MORICONI ET AL.: 'Quantitative gene expression of cytokines in peripheral blood leukocytes stimulated in vitro: modulation by the anti-tumor necrosis factor-alpha antibody infliximab and comparison with the mucosal cytokine expression in patients with ulcerative colitis.' TRANSLATIONAL RESEARCH vol. 150, 2007, pages 223 - 232

### SEQUENCE LISTING

<110> Hitachi Chemical Co., Ltd.
   Hitachi Chemical Research Center, Inc
<120> METHOD FOR CHARACTERIZING HOST IMMUNE FUNCTION BY EX VIVO
   INDUCTION OF OFFENSIVE AND DEFENSIVE IMMUNE MARKERS
<130> HITACHI.097WO
<160> 72
<170> PatentIn version 3.5
<210> 1
   <211> 18
   <212> DNA
   <213> Artificial Sequence
<220>
   <223> Synthesized PCR primer
<220>
   <221> misc_feature
   <223> Sense primer for beta-actin
<400> 1
   cctggcaccc agcacaat 18
<210> 2
   <211> 20
   <212> DNA
   <213> Artificial Sequence
<220>
   <223> Synthesized PCR primer
<220>
   <221> misc_feature
   <223> Antisense primer for beta-actin
<400> 2
   tcatgaggca cacctagccg 20
<210> 3
   <211> 23
   <212> DNA
   <213> Artificial Sequence
<220>
   <223> Synthesized PCR primer
<220>
   <221> misc_feature
   <223> Sense primer for beta-2 microglobulin
<400> 3
   tgactttgtc acagcccaag ata 23
<210> 4
   <211> 20
   <212> DNA
   <213> Artificial Sequence
<220>
   <223> Synthesized PCR primer
<220>
   <221> misc_feature
   <223> Antisense primer for beta-2 microglobulin
<400> 4
   tccaaacttc tacggcgtaa 20
<210> 5
   <211> 20
   <212> DNA
   <213> Artificial Sequence
<220>
   <223> Synthesized PCR primer
<220>
   <221> misc_feature
   <223> Sense primer for CCL2
<400> 5
   ccattgtggc caaggagatc 20
<210> 6
   <211> 19
   <212> DNA
   <213> Artificial Sequence
<220>
   <223> Synthesized PCR primer
<220>
   <221> misc_feature
   <223> Antisense primer for CCL2
<400> 6
   aggtacctgg tggacctgt 19
<210> 7
   <211> 23
   <212> DNA
   <213> Artificial Sequence
<220>
   <223> Synthesized PCR primer
<220>
   <221> misc_feature
   <223> Sense primer for CCL4
<400> 7
   ggtattccaa accaaaagaa gca 23
<210> 8
   <211> 26
   <212> DNA
   <213> Artificial Sequence
<220>
   <223> Synthesized PCR primer
<220>
   <221> misc_feature
   <223> Antisense primer for CCL4
<400> 8
   tcatgcacat actggacctt gacttg 26
<210> 9
   <211> 26
   <212> DNA
   <213> Artificial Sequence
<220>
   <223> Synthesized PCR primer
<220>
   <221> misc_feature
   <223> Sense primer for CCL8
<400> 9
   agagctacac aagaatcacc aacatc 26
<210> 10
   <211> 19
   <212> DNA
   <213> Artificial Sequence
<220>
   <223> Synthesized PCR primer
<220>
   <221> misc_feature
   <223> Antisense primer for CCL8
<400> 10
   tttgccccgt tcctccaga 19
<210> 11
   <211> 28
   <212> DNA
   <213> Artificial Sequence
<220>
   <223> Synthesized PCR primer
<220>
   <221> misc_feature
   <223> Sense primer for CCL20
<400> 11
   gatacacaga ccgtattctt catcctaa 28
<210> 12
   <211> 26
   <212> DNA
   <213> Artificial Sequence
<220>
   <223> Synthesized PCR primer
<220>
   <221> misc_feature
   <223> Antisense primer for CCL20
<400> 12
   acactgtagt tacgatagta gaaagt 26
<210> 13
   <211> 23
   <212> DNA
   <213> Artificial Sequence
<220>
   <223> Synthesized PCR primer
<220>
   <221> misc_feature
   <223> Sense primer for CXCL3
<400> 13
   ggaattcacc tcaagaacat cca 23
<210> 14
   <211> 21
   <212> DNA
   <213> Artificial Sequence
<220>
   <223> Synthesized PCR primer
<220>
   <221> misc_feature
   <223> Antisense primer for CXCL3
<400> 14
   ggtttggctt cagtatcggt g 21
<210> 15
   <211> 21
   <212> DNA
   <213> Artificial Sequence
<220>
   <223> Synthesized PCR primer
<220>
   <221> misc_feature
   <223> Sense primer for CXCL10
<400> 15
   tccacgtgtt gagatcattg c 21
<210> 16
   <211> 21
   <212> DNA
   <213> Artificial Sequence
<220>
   <223> Synthesized PCR primer
<220>
   <221> misc_feature
   <223> Antisense primer for CXCL10
<400> 16
   gtcttagctt ccggtagttc t 21
<210> 17
   <211> 27
   <212> DNA
   <213> Artificial Sequence
<220>
   <223> Synthesized PCR primer
<220>
   <221> misc_feature
   <223> Sense primer for IL8
<400> 17
   tgctaaagaa cttagatgtc agtgcat 27
<210> 18
   <211> 23
   <212> DNA
   <213> Artificial Sequence
<220>
   <223> Synthesized PCR primer
<220>
   <221> misc_feature
   <223> Antisense primer for IL8
<400> 18
   actctcacta actctcacct ggt 23
<210> 19
   <211> 20
   <212> DNA
   <213> Artificial Sequence
<220>
   <223> Synthesized PCR primer
<220>
   <221> misc_feature
   <223> Sense primer for Granzyme B
<400> 19
   gcggtggctt cctgatacaa 20
<210> 20
   <211> 23
   <212> DNA
   <213> Artificial Sequence
<220>
   <223> Synthesized PCR primer
<220>
   <221> misc_feature
   <223> Antisense primer for Granzyme B
<400> 20
   ttcgaggtat ttacagtgga acc 23
<210> 21
   <211> 20
   <212> DNA
   <213> Artificial Sequence
<220>
   <223> Synthesized PCR primer
<220>
   <221> misc_feature
   <223> Sense primer for perforin 1
<400> 21
   tccttggcac ctgtgatcag 20
<210> 22
   <211> 22
   <212> DNA
   <213> Artificial Sequence
<220>
   <223> Synthesized PCR primer
<220>
   <221> misc_feature
   <223> Antisense primer for perforin 1
<400> 22
   actctacgtt ggacttagta cc 22
<210> 23
   <211> 22
<212> DNA
   <213> Artificial Sequence
<220>
   <223> Synthesized PCR primer
<220>
   <221> misc_feature
   <223> Sense primer for TNFSF1
<400> 23
   cagctatcca cccacacaga tg 22
<210> 24
   <211> 22
   <212> DNA
   <213> Artificial Sequence
<220>
   <223> Synthesized PCR primer
<220>
   <221> misc_feature
   <223> Antisense primer for TNFSF1
<400> 24
   tgacagaaga aacctcggaa gc 22
<210> 25
   <211> 22
   <212> DNA
   <213> Artificial Sequence
<220>
   <223> Synthesized PCR primer
<220>
   <221> misc_feature
   <223> Sense primer for TNFSF2
<400> 25
   cgaaggctcc aaagaagaca gt 22
<210> 26
   <211> 20
   <212> DNA
   <213> Artificial Sequence
<220>
   <223> Synthesized PCR primer
<220>
   <221> misc_feature
   <223> Antisense primer for TNFSF2
<400> 26
   tgaaacccta gtaacgggac 20
<210> 27
   <211> 19
   <212> DNA
   <213> Artificial Sequence
<220>
   <223> Synthesized PCR primer
<220>
   <221> misc_feature
   <223> Sense primer for TNFSF5
<400> 27
   ccacagttcc gccaaacct 19
<210> 28
   <211> 24
   <212> DNA
   <213> Artificial Sequence
<220>
   <223> Synthesized PCR primer
<220>
   <221> misc_feature
   <223> Antisense primer for TNFSF5
<400> 28
   ctcataaact taacgttggt ccac 24
<210> 29
   <211> 24
   <212> DNA
   <213> Artificial Sequence
<220>
   <223> Synthesized PCR primer
<220>
   <221> misc_feature
   <223> Sense primer for TNFSF6
<400> 29
   tggcagcatc ttcacttcta aatg 24
<210> 30
   <211> 24
   <212> DNA
   <213> Artificial Sequence
<220>
   <223> Synthesized PCR primer
<220>
   <221> misc_feature
   <223> Antisense primer for TNFSF6
<400> 30
   tctctacaaa acccctgagt aaag 24
<210> 31
   <211> 18
   <212> DNA
   <213> Artificial Sequence
<220>
   <223> Synthesized PCR primer
<220>
   <221> misc_feature
   <223> Sense primer for TNFSF14
<400> 31
   cgt ccgtgtg ctggatga 18
<210> 32
   <211> 22
   <212> DNA
   <213> Artificial Sequence
<220>
   <223> Synthesized PCR primer
<220>
   <221> misc_feature
   <223> Antisense primer for TNFSF14
<400> 32
   cagaatgaag ccccgaaagt ac 22
<210> 33
   <211> 23
   <212> DNA
   <213> Artificial Sequence
<220>
   <223> Synthesized PCR primer
<220>
   <221> misc_feature
   <223> Sense primer for TNFSF15
<400> 33
   tgcgaagtag gtagcaactg gtt 23
<210> 34
   <211> 22
   <212> DNA
   <213> Artificial Sequence
<220>
   <223> Synthesized PCR primer
<220>
   <221> misc_feature
   <223> Antisense primer for TNFSF15
<400> 34
   gttcttcccc tgttcgatta cc 22
<210> 35
   <211> 22
   <212> DNA
   <213> Artificial Sequence
<220>
   <223> Synthesized PCR primer
<220>
   <221> misc_feature
   <223> Sense primer for CD16
<400> 35
   gtttggcagt gtcaaccatc tc 22
<210> 36
   <211> 23
   <212> DNA
   <213> Artificial Sequence
<220>
   <223> Synthesized PCR primer
<220>
   <221> misc_feature
   <223> Antisense primer for CD16
<400> 36
   acgaaccact accatgagga aaa 23
<210> 37
   <211> 24
   <212> DNA
   <213> Artificial Sequence
<220>
   <223> Synthesized PCR primer
<220>
   <221> misc_feature
   <223> Sense primer for IL10
<400> 37
   gccatgagtg agtttgacat cttc 24
<210> 38
   <211> 29
   <212> DNA
   <213> Artificial Sequence
<220>
   <223> Synthesized PCR primer
<220>
   <221> misc_feature
   <223> Antisense primer for IL10
<400> 38
   atcctgtatt taatctccag aggttttag 29
<210> 39
   <211> 24
   <212> DNA
   <213> Artificial Sequence
<220>
   <223> Synthesized PCR primer
<220>
   <221> misc_feature
   <223> Sense primer for TGF-beta 1
<400> 39
   ctgctgaggc tcaagttaaa agtg 24
<210> 40
   <211> 21
   <212> DNA
   <213> Artificial Sequence
<220>
   <223> Synthesized PCR primer
<220>
   <221> misc_feature
   <223> Antisense primer for TGF-beta 1
<400> 40
   tgttaaggac cgctatggag t 21
<210> 41
   <211> 19
   <212> DNA
   <213> Artificial Sequence
<220>
   <223> Synthesized PCR primer
<220>
   <221> misc_feature
   <223> Sense primer for FoxP3
<400> 41
   cacctacgcc acgctcatc 19
<210> 42
   <211> 20
   <212> DNA
   <213> Artificial Sequence
<220>
   <223> Synthesized PCR primer
<220>
   <221> misc_feature
   <223> Antisense primer for FoxP3
<400> 42
   aagtgtgcgt acaaacggaa 20
<210> 43
   <211> 22
   <212> DNA
   <213> Artificial Sequence
<220>
   <223> Synthesized PCR primer
<220>
   <221> misc_feature
   <223> Sense primer for CD25
<400> 43
   cagaagtcat gaagcccaag tg 22
<210> 44
   <211> 20
   <212> DNA
   <213> Artificial Sequence
<220>
   <223> Synthesized PCR primer
<220>
   <221> misc_feature
   <223> Antisense primer for CD25
<400> 44
   tctgtaggca acacgaacgg 20
<210> 45
   <211> 22
   <212> DNA
   <213> Artificial Sequence
<220>
   <223> Synthesized PCR primer
<220>
   <221> misc_feature
   <223> Sense primer for CTLA4
<400> 45
   catgcctcct cttcttcctt ga 22
<210> 46
   <211> 20
   <212> DNA
   <213> Artificial Sequence
<220>
   <223> Synthesized PCR primer
<220>
   <221> misc_feature
   <223> Antisense primer for CTLA4
<400> 46
   atcagtacca ccgtgggagg 20
<210> 47
   <211> 19
   <212> DNA
   <213> Artificial Sequence
<220>
   <223> Synthesized PCR primer
<220>
   <221> misc_feature
   <223> Sense primer for PD-1
<400> 47
   ctcagccgtg cctgtgttc 19
<210> 48
   <211> 23
   <212> DNA
   <213> Artificial Sequence
<220>
   <223> Synthesized PCR primer
<220>
   <221> misc_feature
   <223> Antisense primer for PD-1
<400> 48
   ctcatacggt ggtaacagaa agg 23
<210> 49
   <211> 26
   <212> DNA
   <213> Artificial Sequence
<220>
   <223> Synthesized PCR primer
<220>
   <221> misc_feature
   <223> Sense primer for Arginase
<400> 49
   agacaccaga agaagtaact cgaaca 26
<210> 50
   <211> 19
   <212> DNA
   <213> Artificial Sequence
<220>
   <223> Synthesized PCR primer
<220>
   <221> misc_feature
   <223> Antisense primer for Arginase
<400> 50
   caaagcctga acgagccct 19
<210> 51
   <211> 27
   <212> DNA
   <213> Artificial Sequence
<220>
   <223> Synthesized PCR primer
<220>
   <221> misc_feature
   <223> Sense primer for IL2
<400> 51
   gaactaaagg gatctgaaac aacattc 27
<210> 52
   <211> 24
   <212> DNA
   <213> Artificial Sequence
<220>
   <223> Synthesized PCR primer
<220>
   <221> misc_feature
   <223> Antisense primer for IL2
<400> 52
   aaaacagttt cgtagtagag ttgt 24
<210> 53
   <211> 20
   <212> DNA
   <213> Artificial Sequence
<220>
   <223> Synthesized PCR primer
<220>
   <221> misc_feature
   <223> Sense primer for IL4
<400> 53
   cacaggcaca agcagctgat 20
<210> 54
   <211> 23
   <212> DNA
   <213> Artificial Sequence
<220>
   <223> Synthesized PCR primer
<220>
   <221> misc_feature
   <223> Antisense primer for IL4
<400> 54
   gaacttaagg acaggacact tcc 23
<210> 55
   <211> 23
   <212> DNA
   <213> Artificial Sequence
<220>
   <223> Synthesized PCR primer
<220>
   <221> misc_feature
   <223> Sense primer for Interferon-gamma
<400> 55
   ggagaccatc aaggaagaca tga 23
<210> 56
   <211> 20
   <212> DNA
   <213> Artificial Sequence
<220>
   <223> Synthesized PCR primer
<220>
   <221> misc_feature
   <223> Antisense primer for Interferon-gamma
<400> 56
   aacttacagg ttgcgtttcg 20
<210> 57
   <211> 21
   <212> DNA
   <213> Artificial Sequence
<220>
   <223> Synthesized PCR primer
<220>
   <221> misc**_**feature
   <223> Sense primer for IL17
<400> 57
   gaaatccagg atgcccaaat t 21
<210> 58
   <211> 22
   <212> DNA
   <213> Artificial Sequence
<220>
   <223> Synthesized PCR primer
<220>
   <221> misc_feature
   <223> Antisense primer for IL17
<400> 58
   agttggactt gtaggtattg gc 22
<210> 59
   <211> 18
   <212> DNA
   <213> Artificial Sequence
<220>
   <223> Synthesized PCR primer
<220>
   <221> misc_feature
   <223> Sense primer for GM-CSF
<400> 59
   ggccccttga ccatgatg 18
<210> 60
   <211> 21
   <212> DNA
   <213> Artificial Sequence
<220>
   <223> Synthesized PCR primer
<220>
   <221> misc_feature
   <223> Antisense primer for GM-CSF
<400> 60
   tttgaaggac acgttgggtc t 21
<210> 61
   <211> 23
   <212> DNA
   <213> Artificial Sequence
<220>
   <223> Synthesized PCR primer
<220>
   <221> misc_feature
   <223> Sense primer for IGH@
<400> 61
   cagccggaga acaactacaa gac 23
<210> 62
   <211> 19
   <212> DNA
   <213> Artificial Sequence
<220>
   <223> Synthesized PCR primer
<220>
   <221> misc_feature
   <223> Antisense primer for IGH@
<400> 62
   ctgttctcgt ccaccgtcg 19
<210> 63
   <211> 20
   <212> DNA
   <213> Artificial Sequence
<220>
   <223> Synthesized PCR primer
<220>
   <221> misc_feature
   <223> Sense primer for VEGF
<400> 63
   cgcagctact gccatccaat 20
<210> 64
   <211> 24
   <212> DNA
   <213> Artificial Sequence
<220>
   <223> Synthesized PCR primer
<220>
   <221> misc_feature
   <223> Antisense primer for VEGF
<400> 64
   ctctagctca tgtagaagtt cggt 24
<210> 65
   <211> 19
   <212> DNA
   <213> Artificial Sequence
<220>
   <223> Synthesized PCR primer
<220>
   <221> misc_feature
   <223> Sesne primer for POMC
<400> 65
   acgagggccc ctacaggat 19
<210> 66
   <211> 21
   <212> DNA
   <213> Artificial Sequence
<220>
   <223> Synthesized PCR primer
<220>
   <221> misc_feature
   <223> Antisense primer for POMC
<400> 66
   acaagttttt gcggtagtag t 21
<210> 67
   <211> 19
   <212> DNA
   <213> Artificial Sequence
<220>
   <223> Synthesized PCR primer
<220>
   <221> misc_feature
   <223> Sense primer for GARP
<400> 67
   gacctgatct gccgcttca 19
<210> 68
   <211> 19
   <212> DNA
   <213> Artificial Sequence
<220>
   <223> Synthesized PCR primer
<220>
   <221> misc_feature
   <223> Antisense primer for GARP
<400> 68
   taggaggagt ggtgcgacc 19
<210> 69
   <211> 22
   <212> DNA
   <213> Artificial Sequence
<220>
   <223> Synthesized PCR primer
<220>
   <221> misc_feature
   <223> Sense primer for CD11a
<400> 69
   ggagatcctc gtccaagtga tc 22
<210> 70
   <211> 20
   <212> DNA
   <213> Artificial Sequence
<220>
   <223> Synthesized PCR primer
<220>
   <221> misc_feature
   <223> Antisense primer for CD11a
<400> 70
   gagataccgt cgttgcggag 20
<210> 71
   <211> 28
   <212> DNA
   <213> Artificial Sequence
<220>
   <223> Synthesized PCR primer
<220>
   <221> misc_feature
   <223> Sense primer for CD122
<400> 71
   catatttaca acagagtacc aggtagca 28
<210> 72
   <211> 26
   <212> DNA
   <213> Artificial Sequence
<220>
   <223> Synthesized PCR primer
<220>
   <221> misc_feature
   <223> Antisense primer for CD122
<400> 72
   ggttttcttg ttcttaaaga accatt 26

## Description

### BACKGROUND

### Field of the Invention

The present invention is defined by the claims. Also described herein are markers that can readily be measured and are associated with either the offensive or defensive immune function of a host. More specifically, described herein is the *ex vivo* induction of certain markers associated with either offensive (attacking foreign bodies) or defensive (regulating offensive immune activity) immune function and measurements of their induction as a predictor of host responsiveness to an infection and/or as a method for screening drugs for immuno-modulatory effects. Data generated with respect to gene induction can be used to identify therapies that are specifically tailored to the expression profile of a particular subject.

### Description of Related Art

The immune system comprises a set of diverse proteins, cells, tissues, and processes that protect a host from disease by first identifying and then eliminating pathogens and tumor cells. A primary role of the immune system is to distinguish foreign cells or pathogens from endogenous cells, in other words, distinguishing between "sell" and "non-self." Cells that are endogenous to the host are thought to be recognized as "self" by the expression of Class I Major Histocompatibility Complex (MHC). Those cells without Class I MHC or with reduced levels of expression may be targeted by the immune system as damaged "self" or "non-self" cells. Despite this elegant system, disorders in the immune system can lead to disease, including immunodeficiency, carcinogenesis, or autoimmunity.

White blood cells (WBCs; leukocytes) are the primary functional class of cells in the immune system. While several subtypes of WBCs exist, lymphocytes are one subtype that play an integral role in the immune system defense mechanisms. Natural killer (NK) cells are a specialized type of cytotoxic lymphocyte that are involved in the identification and rejection of tumor cells, virally infected cells, or damaged "self" cells. Cytotoxic T cells are another lymphocyte sub-group that are capable of inducing the death of infected somatic or tumor cells. Once activated, often by cytokines or presentation of a foreign antigen, NK cells and cytotoxic T cells release small granules from their cytoplasm, which contain various proteins and proteases. Certain proteins, such as perforin, induce pore formation in the membrane of a targeted cell, allowing proteases, such as granzymes, to enter the cells and induce the programmed cell death process (apoptosis).

Concurrently with the attack on foreign cells or tumor cells, the immune system also initiates a negative feedback loop to limit the activity of the immune system to shut down the immune system after a successful elimination of foreign cells and also to avoid hyper-responsiveness and possible attacking of "self" cells or other pathways leading to development of auto-immunity. Regulatory T cells actively suppress activation of the immune system and the critical nature of this role is evidenced by the severe autoimmune syndrome that results from a genetic deficiency in regulatory T cells (T-reg). Myeloid-derived suppressor cells (MDSCs) are also involved in the immune down-regulating process, as the MDSCs block the binding of cytotoxic T cells to the foreign proteins expressed on the surface of cells to be targeted and destroyed. Activity of this defensive negative-feedback system may outpace the offensive function of the system, which may increase the probability that tumor cells evade detection and generate a cancerous growth.

Knowing how, and in what capacity, the various cell types are involved in the offensive attack against foreign cells and/or the defensive measures to limit immune system function may provide further insight into the development of cancerous tumors and/or autoimmune diseases. For example, in Mitsuhashi, J Immunol Methods. 2010 Dec 15;363(1):95-100 leukocyte function was investigated by ex vivo stimulation of heparinized whole blood with phytohemagglutinin (T cell stimulator), heat aggregated IgG (IgG Fc receptor stimulator), lipopolysaccharide (toll-like receptor (TLR)-4 stimulator), zymosan (TLR-2 stimulator), monoclonal antibody against T-cell receptor alpha/beta chain, recombinant interleukin-2, and solvent controls, and the subsequent quantification of 32 different leukocyte function-associated mRNAs. Moriconi et al" Transl Res. 2007 Oct;150(4):223-32 quantified mRNA expression of the main acute-phase cytokines and T-cell cytokines in biopsies from patients with established ulcerative colitis (UC) and compared it with that obtained in biopsies from normal controls. Moreover, US 2009/2030064 A1 provides a method of monitoring platelet function in a mammal by passing blood removed from the body of the mammal through a passageway to contact an obstruction or irregularity in the passageway to generate a platelet mass in the passageway, and monitoring the flow or composition of the blood in the passageway to detect the platelet mass. Thus, there exists a need for a diagnostic test to assess both the offensive and defensive immune function in an individual. There also exists a need to rapidly screen drugs for efficacy as immuno-modulating compounds and to indentify therapies for an individual based on that individuals' immune function.

### SUMMARY

Immunity plays a crucial role for the maintenance of good health and in defending against various diseases, ranging from a common cold to life-threatening illnesses.

Although the last several decades have seen great advances in immunology, much of the understanding of how the immune system functions was derived from in vitro experiments or experiments in animal models. Many of the therapies that are administered to patients are simple extrapolations from these experiments and not always effective across a wider variety of patients. Demand is growing for methods of characterizing each patient's immunity or immunological health by clinically applicable techniques.

The present invention is defined by the claims. In more detail, the present invention relates to a method for determining whether an immune-based therapy or a non-immune-based therapy is effective for a cancer patient, wherein the non-immune-based therapy is selected from the group consisting of a radiation, surgery or chemotherapy, said method comprising: Exposing a first aliquot of whole blood from a cancer patient to a solvent comprising an immune stimulating agent selected from zymosan and recombinant interleukin-2 (rIL2); exposing a second aliquot to the solvent not comprising the immune stimulating agent; quantifying the ratio between the amount of mRNA encoding an immune function-related mRNA selected from CD16, FOXP3, and arginase in said first and second aliquot after the exposure, thereby quantifying induction of the immune function-related mRNA; (a) wherein an induction of CD 16 upon exposure to the immune stimulating agent is indicative for an immune based therapy being effective, (b) wherein an induction of FOXP3 upon exposure to the immune stimulating agent is indicative for a non-immune based therapy being effective, or (c) wherein an induction of arginase upon exposure to the immune stimulating agent is indicative for a non-immune based therapy being effective. Also described herein is a method for determining whether a subject's immune function is directed toward offensive or defensive immune function, the method comprising, obtaining a first and a second sample containing leukocytes from a subject, exposing the first sample to an immune stimulating agent in a solvent, wherein the stimulating agent stimulates both offensive and defensive immune function, exposing the second sample to the solvent, quantifying the amount of one or more offensive immune function-related mRNAs in the first and second samples after exposing to the stimulating agent or solvent, thereby quantifying induction of offensive immune function as a ratio between the amount of the offensive immune function-related mRNAs quantified in the first and second samples, quantifying the amount of one or more defensive immune function-related mRNAs in the first and second samples after exposing to the stimulating agent or solvent, thereby quantifying induction of defensive immune function as a ratio between the amount of the defensive immune function-related mRNAs quantified in the first and second samples, wherein significantly more induction of offensive or defensive immune function-indicates that the subject's immune function is directed toward offensive or defensive immune function, respectively.

The samples containing leukocytes may be whole blood samples, which can be optionally heparinized. The stimulation may not need to occur immediately after obtaining the samples. Rather, the whole blood may be stored for up to about 24 hours prior to stimulation. The stored sample may be stored at room temperature. Also the stored sample may be stored in a refrigerated environment (e.g., less than room temperature, for example about 4 degrees Celsius)

Various stimulating agents may be used, including, but not limited to one or more of recombinant interleukin-2, phytohemagglutinin, anti-T-cell receptor antibodies, heat aggregated IgG, lipopolysaccharide, and zymosan. The exposure of the sample to the stimulating agent may be less than 24 hours. Also the exposure may be for between 2 and 6 hours. The exposure may be for about 4 hours.

Functional categories may be used to categorize markers to be studied. The one or more offensive immune function-related mRNAs may be categorized as having immune recruiter function, immune killer function, or immune helper function. The one or more offensive immune function-related mRNAs may have immune recruiter function, and may be selected from the group consisting of CCL2, CCL4, CCL8, CCL20, CXCL3, CXCL10, and Interleukin 8. The one or more offensive immune function-related mRNAs may have immune killer function, and may be selected from the group consisting of granzyme B, perforin, TNFSF1, TNFSF2, TNFSF5, TNFSF6, TNFSF14, and TNFSF15. The one or more offensive immune function-related mRNAs may have immune helper function, and may be selected from the group consisting of interleukin 2, interleukin 4, interferon gamma, and interleukin 17A.

The defensive immune function-related mRNAs are associated with suppression of offensive immune function. The one or more defensive immune function-related mRNAs may be selected from the group consisting of interleukin 10, transforming growth factor-beta, FoxP3, CD25, arginase, CTLA-4, and PD-1.

The methods described herein are optionally used when a subject has a cancer and the subject's immune function is determined as directed toward offensive or defensive immune function so as to identify a potentially efficacious an anti-cancer therapy. In such a use, a determination of the subject's immune function as directed toward offensive immune function indicates the likely efficacy of a cancer immunotherapy regimen. A determination of the subject's immune function as directed toward defensive immune function may indicate the likely efficacy of an anticancer regimen that does not involve an immune-based mechanism of action.

The methods disclosed herein may be used for predicting the efficacy of an anti-cancer therapeutic regimen based on the immune function of a subject, wherein a determination that the subject's immune function is directed toward offensive immune function indicates the likely efficacy of a anti-cancer immunotherapy regimen, and wherein a determination that the subject's immune function is directed toward defensive immune function indicates the likely efficacy of an anticancer regimen that does not involve an immune-based mechanism of action.

Also described herein is a method for determining whether a subject's immune function is directed toward offensive or defensive immune function, the method comprising obtaining first and second samples containing leukocytes from the subject, exposing the first sample to an agent in a solvent, wherein the agent stimulates both offensive and defensive immune function, exposing the second sample to the solvent, incubating the exposed first and second samples, quantifying the amount of one or more defensive immune function-related mRNAs in each of the first and second samples after exposing to the agent or solvent, thereby determining an amount of induction of the defensive-immune function related mRNAs as a ratio between the amount quantified in the first and second samples, quantifying the amount of one or more offensive immune function-related mRNAs in each of the first and second samples after exposing to the agent or solvent, thereby determining an amount of induction of the offensive-immune function related mRNAs as a ratio between the amount quantified in the first and second samples, calculating a ratio between induction of the offensive immune function related mRNAs and the defensive immune function related mRNAs, comparing the calculated ratio with a control ratio derived from a group of control subjects, wherein a significant increase in the calculated ratio over the control ratio indicates the subject's immune function is directed toward offensive immune function and a significant decrease indicates the subject's immune function is directed toward defensive immune function.

The offensive immune function related mRNAs may comprise mRNAs encoding a marker of cytotoxic function. The defensive immune function related mRNAs may comprise mRNAs encoding a marker of myeloid derived suppressor cells, such as for example, arginase or FoxP3.

Also described herein is a method for identifying a drug for administration with a stimulator of both offensive and defensive immune function, wherein the administered drug inhibits one of the offensive or defensive immune functions, the method comprising quantifying the in vitro induction of expression of one or more offensive immune function associated markers in whole blood by measuring the expression of the offensive markers in the presence and absence of the drug (which can be in the presence or absence of the stimulator), quantifying the in vitro induction of expression of one or more defensive immune function associated markers in whole blood by measuring the expression of the defensive markers in the presence and absence of the drug (which can be in the presence or absence of the stimulator), and determining a difference between the induction of offensive immune function associated markers and the induction of defensive immune function associated markers in the presence and absence of the drug, wherein the drug is identified as for administration with the stimulator based on its net effect on the offensive and defensive markers.

The in vitro induction may comprise contacting the whole blood with the stimulator for a period of time sufficient to induce one or more of the offensive and the defensive immune function associated markers. The offensive immune function associated markers may comprise one or more of CD16, granzyme B, TNF-alpha, interferon gamma, and members of the tumor necrosis factor superfamily and the defensive immune function associated markers may comprise one or more of CD25, FoxP3, CTLA4, GARP, IL17, and arginase. The stimulator of offensive and defensive immune function may be interleukin 2. The drug that wither offensive or defensive immune function may induce expression of one or more offensive immune function associated markers to a greater degree that the drug induces the expression of one or more defensive immune function associated markers (e.g., it preferentially induces offensive function). The increase is optionally determined relative to a control sample that is stimulated with a solvent, which is the solvent used to deliver the drug. The administration of the drug is optionally prior to or concurrent with administration of the stimulating agent.

Also described herein is a method of characterizing both offensive and defensive immune function comprising obtaining at least two aliquots of heparinized whole blood, exposing the first aliquot to an agent that stimulates both offensive and defensive immune function, exposing the second aliquot to the solvent of the agent, incubating these aliquots for less than 24 hours, quantifying the amount of one or more mRNAs encoding TNFSF, granzyme B, perforin, CD16, INFgamma, or other genes representing the cytotoxic functions of leukocytes quantifying the amount of one or more mRNAs encoding FoxP3, CD25, or other genes representing the marker of regulatory T cells, calculating the ratio between expression of the markers of cytotoxic functions and the markers of regulatory T cells in both the first and second aliquots, and comparing these ratios with that derived from a group of control subjects.

The method optionally further comprises obtaining two additional aliquots of heparinized whole blood, exposing the first additional aliquot to an agent that stimulates both offensive and defensive immune function, exposing the second additional aliquot to the solvent of the agent, incubating these aliquots for less than 24 hours, quantifying the amount of one or more mRNAs encoding arginase or other genes representing the marker of myeloid derived suppressor cells, calculating the ratio between expression of the markers of cytotoxic functions and the markers of myeloid derived suppressor cells in both the first and second additional aliquots, and comparing these ratios with that derived from a group of control subjects.

### BRIEF DESCRIPTION OF THE FIGURES

Figures 1A-1D depicts the change over time in mRNA levels encoding various immune markers following IL-2 stimulation.
Figure 2 depicts the dose response induction of immune markers to IL-2. A "*" indicates statistical significance (p<0.05) compared with solvent control.
Figures 3A-3J depicts the data used for drug screening.
Figures 4A-4J depict data related to induction of Offensive or Defensive immune markers used in drug screening.
Figures 5A-5D depict a rapid high throughput protocol.

### DETAILED DESCRIPTION

Methods are provided herein for the *ex vivo* characterization of the offensive and defensive immune response of a host. As used herein, the term "offensive" shall refer to the overall immune response and cellular components associated therewith mounted against an infection, a foreign pathogen, a tumor or the like. In some instances the term "killer" is used interchangeably with "offensive." As used herein, the term "defensive" shall refer to the overall immune response and cellular components associated therewith that serve to limit the activity of the activated offensive immune system. In some instances the term "suppressor" is used interchangeably with "defensive."

The methods may involve collection of peripheral whole blood from a host and the use of a stimulating agent or agents to induce one or more of a panel of markers associated with the offensive or defensive immune response. Isolated leukocytes may optionally be used. Measurement of the mRNA encoding one or more of each of the offensive or defensive markers may be used to characterize the overall immune response of a host. The characterization of the expression of such offensive or defensive markers may be used to screen drugs for potential efficacy as either an immunosuppressant drug or an anti-cancer drug (e.g., immuno-modulating drugs).

The immune system comprises a variety of cell types having a variety of functions, which work together in concert to mount an attack on foreign bodies, thereby protecting the host from infection, tumorogenesis, etc. The main categories of function include, but are not limited to, recruitment function, killer function, suppressor (of killer) function, and helper function, as well as a variety of auxiliary functions, e.g., antigen presentation, regulation of angiogenesis, pain modulation etc.

Recruitment function is essential for the proper function of the immune system. In the event of an infection, tumor formation, etc., immune cells must be mobilized from various parts of the body, including the whole blood, bone marrow, and lymphatic system, among others, in order to properly recognize and defend the host from an immune challenge. Chemokines may function to recruit other immune cells to the local area of inflammation or tumor formation. In essence, having an host of cells that can kill or disable unwanted foreign cells is useless if those cells are not properly instructed on where to go to function. Recruiter function may be provided by chemokines or other chemotactic molecules. Chemokines of a particular motif function to recruit other immune molecules. For example, CCL molecules, such as CCL-2, CCL-4, CCL-8, or CCL-20 may be involved in recruiting other immune cells. CXCL molecules, such as CXCL-3 or CXCL-10 are involved. Also other chemokine effectors, whether C-C or C-X-C motif or another variety, may be involved.

Once the recruiter cells have brought other types of immune cells to the proper location, the other types of cells can perform their designated function may be to kill the target cell(s). The killing function may be realized by induction of apoptosis in the target cell. For example, when the target is a tumor, one or more cells having killer function (e.g., expressing certain molecules involved in apoptosis) are recruited to the target site. Such killer cells may express one or more of molecules such as Granzyme B, perforin, TNFSF1 (lymphotoxin), TNFSF2 (TNF-alpha), TNFSF 5 (CD40 ligand), TNFSF6 (Fas ligand), TNFSF14 (LIGHT), TNFSF 15 (TL1A), and/or CD16. As such, the recruitment of these cells to the target site initiates a cascade that results in the destruction of the target cells, and thus realizes the goal of the offensive immune system, e.g., destruction and/or removal of a foreign body or cell.

As discussed herein, the defensive immune system comprises a series of signals and molecules that function to limit the activity of the offensive immune system (e.g., prevent overactive offensive function, which could lead to autoimmune disorders). Cells having defensive function can be recognized by markers including, but not limited to, IL10, TGF-beta, (forkhead box p3) FoxP3, CD25, arginase, CTLA-4, and /or PD-1. Such cells are an important balance on activity of the offensive immune system and are important to ensure proper overall immune function.

Additional cells types are involved in the functioning of both the offensive and defensive immune system. Helper T-cells (Th cells) are a sub-group of lymphocytes function to establish and maximize the capabilities of the immune system. Unlike the cells described above, Th cells lack cytotoxic or phagocytic activity. Th cells are, however, involved in activating and directing other immune cells such as the cytotoxic T cells (e.g., the killer cells described above). Th cells are divided into two main subcategories (Th1 or Th2) depending on, among other factors, what cell type they primarily activate, what cytokines they produce, and what type of immune stimulation is promoted. For example, Th1 cells primarily partner with macrophages, while Th2 cells primarily partner with B-cells. Th1 cells produce interferon-gamma, TNF-beta, and IL-2, while Th2 cells product IL1, IL5, IL6, IL10 and IL13. Markers of the subsets of Th cells are known and can be used to identify the induction of certain Th cell subtypes in response to stimulation. For example, the induction of IL2 or IFNG represent responses to stimulation by Th1 cells, while induction of IL4 or IL10 represent responses to stimulation by Th2 cells. Other subtypes, such as Th17 are represented by other markers, such as IL17 (see e.g., Tables 5 and 6).

Finally, a variety of other functions are useful to study when characterizing immune status or function of a subject. For example, antigen presentation (measured by GMCSF), proliferation of B-cells (measured by IGH2), angiogenesis, which is often occurs in tumor formation due to increased blood flow demands (measured by VEGF), and pain (measured by POMC). These general categories described above can be used to categorize interpret data generated by stimulation of whole blood in order to characterize a subject's immune response, which is described in more detail below.

Building on the general functional categories described above, offensive immune function, such as the function of NK cells and cytotoxic T cells is important for destruction of cancerous cells and combating infections and/or inflammation. Due to their ability to potentially kill both unwanted target cells as well as normal endogenous cells, NK cells possess two types of surface receptors, activating receptors and inhibitory receptors. Together, these receptors serve to balance the activity of, and therefore regulate, the cytotoxic activity of NK cells. Activating signals are required for activation of NK cells, and may involve cytokines (such as interferons), activation of FcR receptors to target cells against which humoral immune responses have been mounted, and/or foreign ligand binding to various activating NK cell surface receptors. Targeted cells are then destroyed by the apoptotic mechanism described above.

Similarly, cytotoxic T cells also require activation, thought to be through a two signal process resulting in the presentation of a foreign (e.g., non-self) antigen to the cytotoxic T cells. Once activated, cytotoxic T cells undergo clonal expansion, largely in response to interleukin-2 (IL-2), a growth and differentiation factor for T cells. Cytotoxic T cells function somewhat similarly to NK cells in the induction of pore formation and apoptosis in target cells.

In addition to the immune attack on foreign cells, defensive immune function develops, which is believed to be moderated by T-reg and MDSCs, and inhibits offensive immune function. Developing in the thymus, many T-reg express the forkhead family transcription factor FoxP3 (forkhead box p3). FoxP3 expression appears to be required for T-reg development and population expansion and may also be a controlling factor in a genetic program defining the T-reg fate. In many disease states, particularly cancers, alterations in T-reg numbers, particularly those T-reg expressing Foxp3, are found. For example, patients with tumors have a local relative excess of Foxp3 positive T cells which inhibits the body's ability to suppress the formation of cancerous cells.

MDSCs also are effectors of the defensive immune response. While MDSCs do not appear to destroy offensive T cells, they do alter how cytotoxic T cells behave. MDSCs secrete arginase (ARG), a protease that breaks down the amino acid arginine. Lymphocytes, including cytotoxic T cells and NK cells are indirectly dependent on arginine for activation. Thus, the secretion of ARG by MDSCs limits the activation of NK cells and cytotoxic T cells, thereby promoting the defensive immune response.

However, as a result of this self-limiting regulation by T-reg and MDSCs, defensive immune function has the potential to become the dominant scheme in a local tissue environment. As a result, the stimulation of offensive immune function (as developed by cancer vaccine treatment or adaptive immunotherapy) may fail to function sufficiently to completely eradicate tumor cells. As a result, a tumor cell may escape the immune system and metastasize into a tumor.

Thus, the characterization of offensive and defensive immune function in an individual may be critically important, as domination by the offensive system may promote auto-immunity and domination by the defensive system may be pro-cancerous. Furthermore, the expression profiles of offensive and defensive-associated immune markers may be useful to screen drugs for their efficacy as anti-cancer medications or immunosuppressant agents.

For example, the expression profiles of various markers can be used to identify therapies of a particular variety that may be most efficacious for a particular subject. The stimulation of whole blood by a variety of markers enables a determination of what cell types are responsive to a particular type of stimulus. As discussed herein, stimulants include, but are not limited to, IL2 (a general cellular immune modulator), PHA (a general T-cell modulator), anti-T-cell receptor antibodies (specific stimulator of T-cells), HAG (stimulant for leukocytes having the FcR receptor), and LPS or zymosan (activators of the Toll-like receptor, associated with general bacterial immune responses). When used to stimulate whole blood and measure the induction of expression of various markers from the general functional categories described herein (e.g., recruiter or killer), the pattern of induction can be used to identify potential therapies that are particularly effective for a given subject. As a non-limiting example, if a sample of blood from a patient having cancer is stimulated and one or more markers of offensive (e.g., killer) function are increased, this initially suggests that an immunotherapy based cancer treatment may be effective for this subject. The strength of the initial suggestion may be increased based on the expression levels of markers from other categories. For example, if the induction of one or more offensive markers is associated with a stable (e.g., little or no change) in suppressor function, this further suggests that an immunotherapy based cancer treatment would likely be effective, given the increase in offensive function without a coordinate increase in defensive function that would limit the efficacy of the offensive arm of the immune system. In other words, the increased gap in function, as represented by expression levels, between the offensive and defensive immune systems indicates that a therapy exploiting the increased offensive activity would be effective. On the other hand, for example, if stimulation of a patient's whole blood yields an increase in offensive markers as well as in defensive markers (e.g., no net change in function between the systems despite the increase), these results would suggest a non-immune-based therapy may be more efficacious for this subject. This is because the lack of a net change in function between the offensive and defensive systems suggests that both systems are upregulated in function, and that the offensive system is not likely to be sufficiently dominant to render an immune-based therapy particularly effective. In such a context, therapies such as radiation, surgery, or chemotherapy may be more effective. Additional information from expression levels of markers in other functional categories may support or refute other data related to the potential efficacy of a particular type of therapy. For example, stimulation of the whole blood of a cancer patient that results in increased recruiter marker expression in conjunction with increased offensive marker expression and little or no change in defensive marker expression further supports the potential efficacy of an anti-cancer therapy that is immune-based. This is because the increased recruiter activity is likely to further enhance the increased offensive function by enabling the cells of the offensive immune system to be recruited, for example, more quickly, in greater numbers, and/or over a longer period of time. Expression changes in functional categories may be evaluated and used to determine a potentially optimal therapy, while also, individual markers from within a category may be used to determine a potentially optimal therapy.

Numerous methods for assessing the expression of markers of interest are available. For example, flow cytometric analysis may allow the identification of NK cells, cytotoxic T cells, T-reg, and MDSCs by staining appropriate marker proteins. However, such an assay system is not capable of analyzing each cell's function. Therefore, several methods involve the *ex vivo* induction and measurement of offensive and defensive-associated immune response mRNAs as a diagnostic test to characterize a patient's overall immune response.

Moreover, many prior experiments directed to determining expression of immune system activity and/or markers of activity have been done in isolated leukocyte preparations. Such isolated populations are often preferred because the variety of lymphocytes in whole blood may preclude detection of induction of a specific, mRNA in a small subset of lymphocytes. Moreover, with numerous complex biochemical interactions between the multiple types of lymphocytes, there is the possibility that use of a whole blood preparation inhibits or modifies the induction and measurement reactions. Furthermore, stimulatory agents, such as IL-2 and zymosan may interact with plasma proteins or plasma factors, and thereby exhibit decreased or reduced induction activity. However, when used as presented herein, whole blood unexpectedly produces reproducible, accurate, and physiologically relevant results that allow the characterization of both offensive and defensive immune markers and screening of drugs of immune-modulating efficacy.

Whole blood may be collected from mammals, preferably humans. Preferably, the collected whole blood is heparinized upon collection. The collected whole blood may be stored at 4° C until the stimulation protocol (described below). Preferably whole blood is employed, or blood cells separated from plasma may also be used, as well as isolated leukocyte preparations.

In the method described herein, the blood may be aliquoted into small volumes (approximately 40-100 microliters (µL)), each of which is treated (i.e., induced or stimulated) with either a stimulating agent carried in a solvent or a control agent. The control agent may induce little or no response in the blood samples. The control agent may be the same solvent used to carry the stimulating agent. The control agent may be phosphate-buffered saline (PBS), while also, the control agent may be dimethyl sulfoxide (DMSO). Recombinant IL-2 (rIL-2) may be used as a stimulating agent for both offensive and defensive immune markers. IL-2 is often used clinically as an agent to augment offensive immune response. However, this clinical effort also fails on some occasions, as IL-2 can also simultaneously upregulate the defensive immune system. Thus, in order to develop a more complete analysis of both major portions of the immune system, IL-2 is a preferred stimulatory agent herein. Also, zymosan, a ligand of the toll-like receptor type 2 (TLR-2), may be used as a stimulating agent for both offensive and defensive markers. In addition, other known immune-stimulating agents may used. Yet further, agents known to stimulate particular offensive and/or defensive immune markers may be used.

The stimulating agents may induce the expression of one or more offensive or defensive immune markers, as measured by the amount of mRNA encoding said markers. Offensive markers include, but are not limited to, CD16 (surface marker of NK cells); granzyme B (inducer of rapid apoptosis); perforin (cytolytic protein that functions to lyse cells); TNFSF1 (lymphotoxin, functions to enhance phagocytic cell binding to a target cell); TNFSF2 (TNF-alpha; inducer of slow apoptosis); TNFSF5 (CD40 ligand, operates to activate antigen presenting cells and macrophages); TNFSF6 (Fas ligand, inducer of apoptosis); TNFSF14 (LIGHT; induces T-cell proliferation and apoptosis of tumor cells); TNFSF15 (inducer of apoptosis). Defensive immune markers include, but are not limited to IL10 (down-regulator of Th1 cytokines); TGF-beta (blocks lymphocyte activation); CD25 (surface marker of T-reg); FoxP3 (T-reg marker); CTLA4 (Cytotoxic T-lymphocyte antigen); GARP (glycoprotein A repetitions predominant); IL17 (putative negative regulator of T cell activation); ARG (arginase, marker of MDSC); and PD-1 (programmed death 1, negative regulator of T-cell responses).

In the method described herein, induction of offensive or defensive immune markers may be accomplished by mixing a small aliquot of a blood sample with either a control agent in triplicate, or one of the stimulating agents in triplicate. The mixture is then incubated at 37° C, for a period of time sufficient for induction of the offensive or defensive immune markers to occur. The incubation time may be approximately 4 hours. The incubation time may be greater than 4 hours. The incubation time may be approximately 24 hours. The incubation time may be less than 4 hours. After the appropriate incubation period, all the blood samples are stored at -80° C until further analysis.

A small volume of the previously stimulated blood from each sample may be processed to allow determination of the levels of mRNA encoding one or more offensive or defensive immune markers in the blood. The levels of mRNA encoding one or more offensive or defensive immune markers might change significantly in response to the stimulating agent. To determine these mRNA levels, the erythrocytes and blood components other than leukocytes are removed from the blood sample. The leukocytes may be isolated using a device for isolating and amplifying mRNA. Embodiments of this device are described in more detail in United States Patent Application Nos.: 10/796,298, 11/525,515, 11/376,018, 11/803,593, 11/803,594, and 11/803,663.

In brief, the device described herein may comprise a multi-well plate that contains a plurality of sample-delivery wells, a leukocyte-capturing filter underneath the wells, and an mRNA capture zone underneath the filter which contains immobilized oligo(dT). The device may also contain a vacuum box adapted to receive the filter plate to create a seal between the plate and the box, such that when vacuum pressure is applied, the blood is drawn from the sample-delivery wells across the leukocyte-capturing filter, thereby capturing the leukocytes and allowing non-leukocyte blood components to be removed by washing the filters. Other means of drawing the blood samples through out of the sample wells and through the across the leukocyte-capturing filter, such as centrifugation or positive pressure, may be used. In a preferred device, leukocytes are captured on a plurality of filter membranes that are layered together. The captured leukocytes may then be lysed with a lysis buffer, thereby releasing mRNA from the captured leukocytes. The mRNA is then hybridized to the oligo(dT)-immobilized in the mRNA capture zone. Further detail regarding the composition of lysis buffers that may be used herein can be found in United States Patent Application No.:11/376,018. cDNA may be synthesized from oligo(dT)-immobilized mRNA. The cDNA may then be amplified using real time PCR with primers specifically designed for amplification of infection-associated markers. Primers that are used in such embodiments are shown in Table 1. Further details about the PCR reactions used herein are also found in United States Patent Application No.:11/376,018.

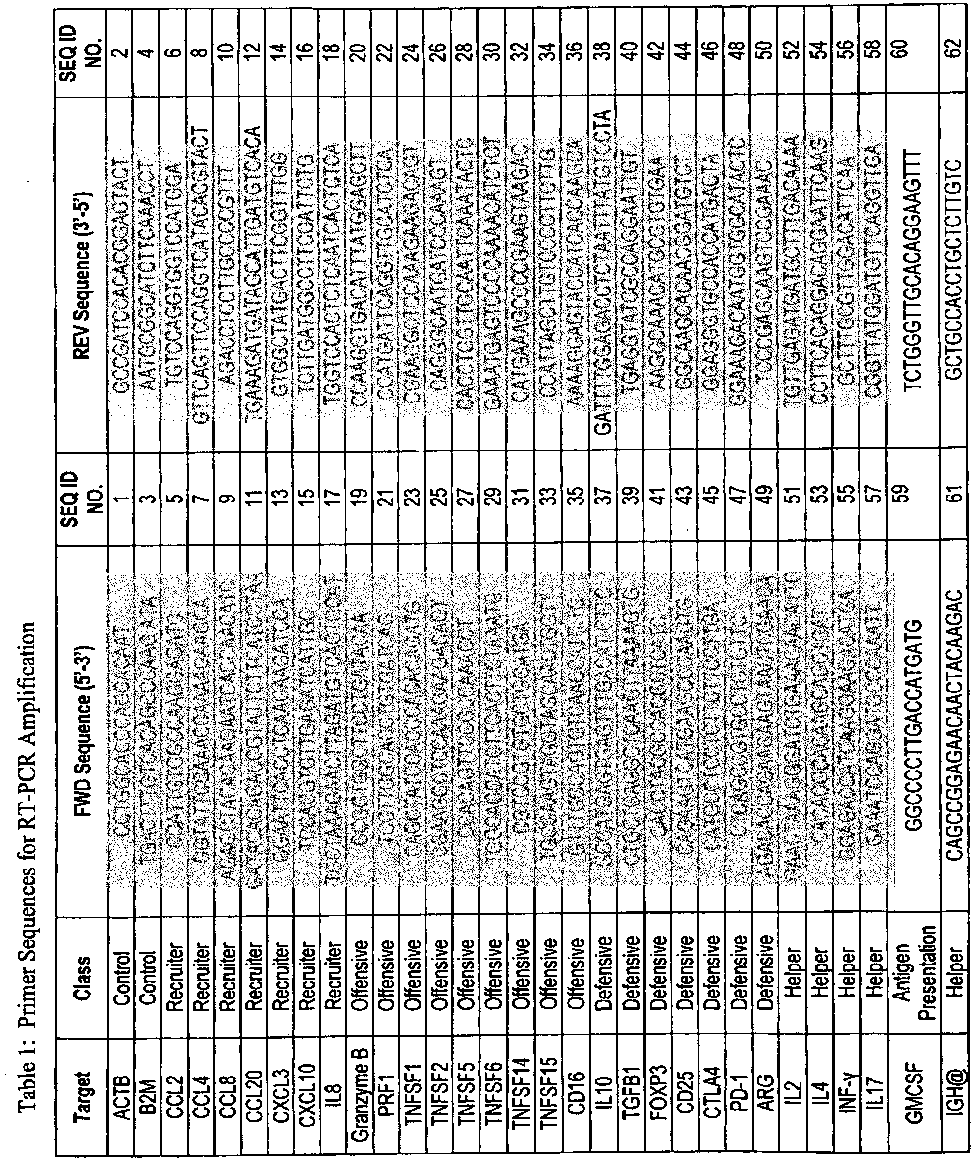

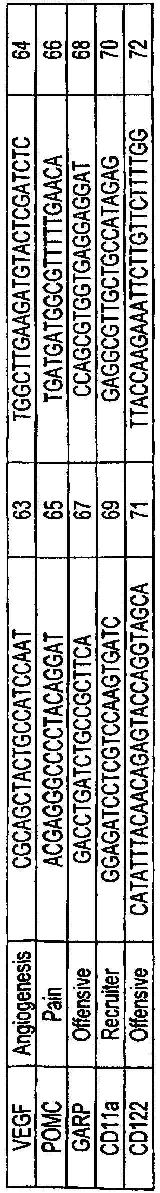

After the completion of PCR reaction, the mRNA (as represented by the amount of PCR-amplified cDNA detected) for one or more offensive or defensive immune markers is quantified. Quantification may be calculated by comparing the amount of mRNA encoding an offensive or defensive immune marker to a reference value. The reference value may be expression level of a gene that is not induced by the stimulating agent, e.g., a house-keeping gene. Beta-actin may be used as the reference value. Numerous other house-keeping genes that are well known in the art may also be used as a reference value. Also a house keeping gene may be used as a correction factor, such that the ultimate comparison is the induced expression level of an offensive or defensive immune marker as compared to the same marker from a non-induced (control) sample. Still further, the reference value may be zero, such that the quantification of the offensive or defensive immune markers is represented by an absolute number. A ratio comparing the expression of one or more offensive immune markers to one or more defensive immune markers may be made.

Offensive or defensive immune marker expression may be measured in the presence of a drug (either a putative anti-cancer or immunosuppressant drug) both in the presence and in the absence of a stimulating agent. Here, the expression profiles may be used to predict the efficacy of a drug compound as an effective anti-cancer drug or as an effective immunosuppressant drug. A drug compound might induce the expression of an offensive immune marker but not a defensive marker, which would promote the offensive immune system overall, thus making that drug compound a putative anti-cancer therapeutic. Likewise, a drug may inhibit one or more defensive markers, which would promote the offensive immune system overall, thus making that drug compound a putative anti-cancer therapeutic. Here, a drug that blocks a defensive immune marker, and hence reduces the defensive immune component could be co-administered with a therapeutic agent known to stimulate the offensive immune system (such as IL-2), thereby providing an enhance offensive immune response and increased likelihood of tumor cell elimination.

In contrast, a drug compound may induce the expression of a defensive immune marker but not an offensive marker, which would promote the defensive immune system overall, thus making that drug compound a putative immunosuppressant. Likewise, a drug may inhibit one or more offensive markers, which would promote the defensive immune system overall, thus making that drug compound a putative immunosuppressant.

A drug compound may not induce either offensive or defensive marker, or may induce both. Further dose-response study may be performed to determine if a particular dose or exposure time categorizes a drug as a putative anti-cancer drug or putative immunosuppressant.

### EXAMPLES

Specific embodiments will be described with reference to the following examples which should be regarded in an illustrative rather than a restrictive sense.

### Example 1- Characterization of Offensive and Defensive Immune Markers in Whole Blood Samples

Whole blood samples were collected from healthy human adults. Blood samples were heparinized when collected and placed into several individual tubes for an induction time course study. Eighteen equal volume aliquots from each tube were then stimulated with either phosphate buffered saline (PBS), rIL-2 at 100 ng/mL, or phytohemagglutinin (PHA). The aliquots were incubated at 37°C for 0, 1, 2, 4, 8, or 20 hours. After incubation, samples were stored at -80°C until analysis. Each sample was stimulated and analyzed in triplicate. mRNA encoding beta-actin, IL-2, IL-2 receptor type a (CD25) and IL-2 receptor type b (CD 122) were measured according to the methods described in Mitsuhashi M, et al., Clin Chem 52:634-642 (2006).

Briefly, 96-well filterplates were assembled with leukocyte reduction membranes (Leukosorb; Pall) and placed over oligo(dT)-immobilized collection plates. 150 µL of 5 mmol/L Tris (pH 7.4) was applied to wet the filter membranes. After centrifugation at 120g for 1 min at 4 °C to remove the Tris solution from the membranes, 50 µL of the stimulated whole blood samples was applied to each well and immediately centrifuged at 120g for 2 min at 4°C. The wells were then washed once with 300 µL of phosphate-buffered saline. After centrifugation at 2000g for 5 min at 4 °C to remove the saline solution, 60 µL of stock lysis buffer [5 g/L *N-*lauroylsarcosine, 4x standard saline citrate, 10 mmol/L Tris-HCl (pH 7.4), 1 mmol/L EDTA, 1 mL/L IGEPAL CA-630 (substitute of NP-40), 1.79 mol/L guanidine thiocyanate (all from Sigma)], supplemented with 10 mL/L 2-mercaptoethanol (Bio-Rad), 0.5 g/L proteinase K (Pierce), 0.1 g/L salmon sperm DNA (5 Prime Eppendorf/Brinkman), 0.1 g/L *Escherichia coli* tRNA (Sigma), 5 nmol/L each of the specific reverse primers, and 10¹⁰ molecules/L of synthetic RNA34 (as external control), was added to each well of the filterplates. The plates were then incubated at 37 °C for 10 min, placed over oligo(dT)-immobilized collection microplates (GenePlate; RNAture), and centrifuged at 2000g for 5 min at 4 °C. After overnight storage at 4°C, the microplates were washed 3 times with 100 µL of plain lysis buffer and then 3 times with 150 µL of wash buffer [0.5 mol/L NaCl, 10 mmol/L Tris (pH 7.4) 1 mmol/L EDTA] at 4°C.

cDNA was synthesized directly in each well by addition of 30 µL of buffer containing 1x reverse transcription buffer [50 mM KCl, 10 mM Tris-HCl (pH 8.3), 5.5 mM MgCl₂, 1 nL/µL Tween 20], 1.25 mM each deoxynucleoside triphosphate, 4 units of rRNasin, and 80 U of MMLV reverse transcriptase (Promega; without primers) and incubation at 37 °C for 2 h. From each 30 µL reaction, 4 µL of cDNA was transferred directly to 384-well PCR plates, and 5 µL of TaqMan universal master mixture (Applied Biosystems) and 1 µL of 5 µM each of the forward and reverse primers for an infection-associated marker or beta-actin (see Table 1) were added. Primer sequences used are shown in Table 1 above. Primer sequences for ACTB (β-actin) were also published previously (Mitsuhashi M, et al. Pharm Res. 25:1116-1124, 2008). PCR was carried out in a PRISM 7900HT (Applied Biosystems), with 1 cycle of 95 °C for 10 min followed by 45 cycles of 95 °C for 30 s, 55 °C for 30 s, and 60 °C for 1 min. Each gene was amplified in separate wells. The cycle threshold (Ct), i.e., the cycle at which certain amounts of PCR products (based on fluorescence) were generated, was determined with analytical software (SDS; Applied Biosystems). The Ct of each mRNA was subtracted with that of ACTB to calculate ΔCt, and %ACTB was calculated by 2^{-ΔCt} x 100.

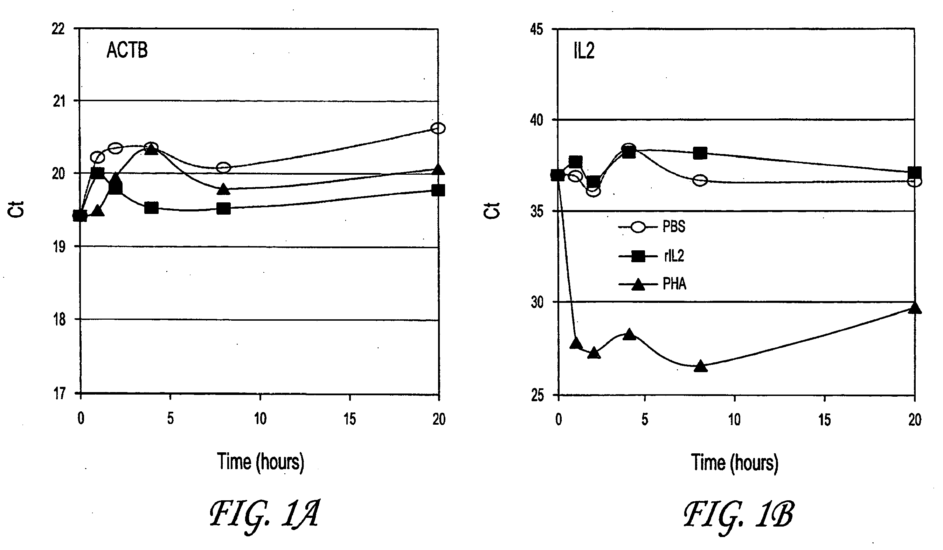

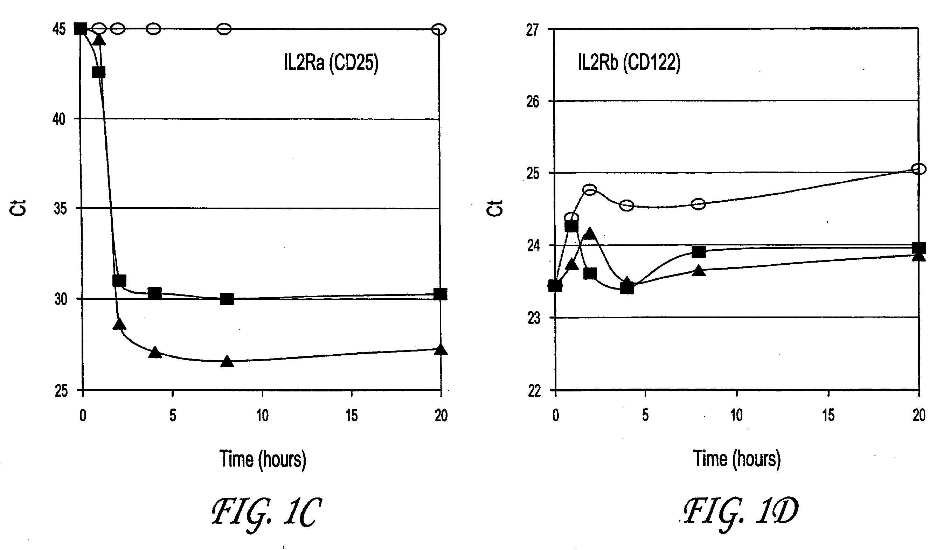

As shown in Figure 1A, stimulation of whole blood does not induce beta actin expression, regardless of the stimulating agent used. Figure 1B serves as a positive control for the stimulation assay, as IL-2 is not self-inducing, but PHA, a known stimulator of inflammatory and immune responses robustly increased the expression of IL-2. Figure 1C demonstrates that the induction of a defensive immune marker CD25 occurs subsequent to stimulation by either rIL-2 or PHA (an inducer of IL-2). This also demonstrates the cascade effect that IL-2 stimulation has on both the offensive and defensive aspects of the immune system.

Additional aliquots of the whole blood samples were used for a dose-response study. Seven aliquots were stimulated for four hours with rIL-2 at 100 ng/mL. After incubation, samples were stored at -80°C until analysis. Each sample was stimulated and analyzed in triplicate. mRNA encoding beta-actin, IL-2, IL-2 receptor type a (CD25), IL-2 receptor type b intronic sequence (CD 122), IL-2 receptor type b exon sequence (CD 122), granzyme B, TNF-alpha, and interferon-gamma were measured according to the methods described above.

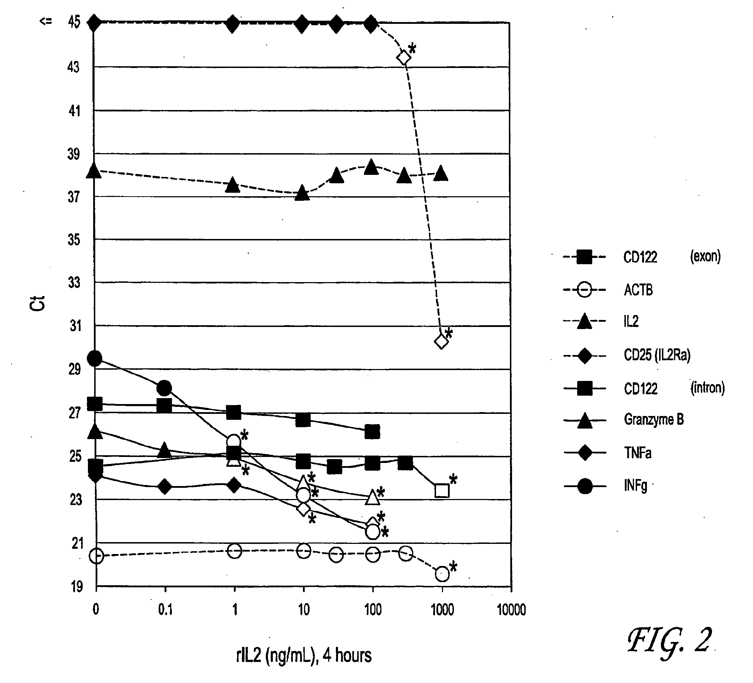

As shown in Figure 2, offensive and defensive markers may respond differently to a given concentration of stimulatory agent. Based on the effect on induction of offensive markers and the time course depicted in Figure 1, stimulation with 100ng/mL rIL-2 for 4 hours were the conditions chosen for a panel of stimulation experiments. It shall be appreciated that greater or lesser concentrations of a stimulatory agent may be used in other embodiments. Likewise, greater or lesser times for stimulation may be used, for example 1-2 hrs, 2-4 hours, 4-6 hours, 6-8 hours, 8-10 hours, 10-12 hours, 12-18 hours, 18-24 hours, and overlapping ranges thereof.

Induction of expression of a panel of offensive or defensive markers was studied in response to stimulation of whole blood by rIL-2 or zymosan. Samples were generally prepared and stimulated as described above, with rIL-2 used at 100ng/ml and zymosan used at 1.5 mg/mL and stimulation was for four (4) hours at 37° C.

As shown in Table 2, stimulation with rIL-2 induced statistically significant (P<0.05) increases in all offensive markers tested in the panel. While TNFα, CD16, and Granzyme B were induced approximately 4-8 fold over control samples, IFNγ was induced 260 fold over control samples. rIL-2 also induced statistically significant increases in several defensive markers, namely FoxP3, CD25, and IL17. Zymosan induced a statistically significant increase in arginase. These results demonstrate that the methods of certain embodiments of the invention as disclosed herein unexpectedly allow the detection of increased expression of both offensive and defensive markers of immune function, in contrast to the suggestion and conventional wisdom in the related art that isolated leukocyte preparations are needed. These data indicate that stimulation conditions used in several embodiments induce the expression of various offensive and defensive markers well above the threshold for detection (background noise) of the assay and provide a rapid, simple, and reliable method of characterizing the offensive and defensive immune response of an individual.

**Table 2. Leukocyte Function by Ex Vivo Stimulation**

| | | **rIL-2 (100ng/mL)** | | | **Zymosan (TLR-2; 1.5 mg/mL)** | | |
|---|---|---|---|---|---|---|---|
| | **mRNA** | **Avg.** | **S.D.** | **p-value** | **Avg.** | **S.D.** | **p-value** |
| **Control** | ACTB | 0.9088 | 0.0756 | 0.247 | 0.63 | 0.011 | 2 x 10⁻⁶* |
| **Offensive** | TNFα | 4.73 | 0.431 | 5 x 10⁻⁴* | 11 | 0.886 | 8 x 10⁻⁵* |
| | CD16 | 6.1303 | .03566 | 5 x 10⁻⁴* | | | |
| | Granzyme B | 8.2773 | 1.5272 | 2 x 10⁻⁴* | | | |
| | IFNγ | 260.48 | 78.401 | 2 x 10⁻⁵* | 5.948 | 6.978 | n.s. |
| **Defensive** | FoxP3 | 3.123 | 0.8372 | 0.029^{*} | | | |
| | CD25 | 16.035 | 0.999 | 5 x 10⁻⁴* | | | |
| | CTLA4 (1^{st} Primer) | 1.0843 | 0.1799 | n.s. | | | |
| | GARP | 0.7404 | 0.14 | n.s. | | | |
| | IL17 | 18.315 | 7.2536 | 0.005^{*} | | | |
| | ARG | | | | 3.618 | 0.846 | 0.005^{*} |

| | | | | | | | |
|---|---|---|---|---|---|---|---|
| * Fold increases >2 are statistically significant based on a p-value of <0.05 | | | | | | | |

### Example 2- Induction of Offensive and Defensive Immune Markers as Method of Drug Screening

Several embodiments of the present invention are used to screen candidate drug compounds based on the ability of the compounds to induce expression of offensive or defensive immune markers. In some embodiments, such a drug screening assay would increase the efficiency of identifying a compound that could be co-administered with IL-2 in an anti-cancer clinical setting, the compound ideally blocking increases in expression of defensive immune markers. Thus, IL-2 stimulation would induce offensive immune responses to putative cancer cells, and the candidate compound would block the IL-2 induced negative feedback defensive immune responses, thereby limiting the self-induced down regulation of the offensive response. In this manner, the offensive immune response is effectively potentiated by the lack of (or reduction of) defensive immune responses.

Similarly, several embodiments are used to screen for potential immunosuppressant compounds. Potential efficacious compounds are those that inhibit the induction of offensive immune markers and do not effect (or increase) defensive immune markers, thereby promoting the activity of the defensive immune system over those of the offensive system. Such a response would allow for suppression of the endogenous immune response and potentially provide benefit to transplant patients or patients suffering from autoimmune disorders.

While certain compounds may exhibit likely efficacy for suppressing (or stimulating) offensive or defensive immune function, it shall be appreciated that potential efficacy may not be shown at a single dose tested. Thus, in some embodiments, dose-response testing is also performed to determine whether a potential compound with limited efficacy at a first dose has enhanced efficacy (as offensive or defensive immune stimulator) at different doses, which may be higher or lower than the first concentration.

To screen potential compounds, heparinized whole blood was preincubated with various potential immune inhibitor compounds (all 10 mM in final concentration) or solvent control (DMSO) for 1 hour in a single tube for each treatment, then stimulated with PBS or rIL-2 (100 ng/ml in final concentration) in triplicate for additional 4 hours. mRNA was then quantified as described above. Potential immune inhibitor compounds tested are shown in Table 3.

**Table 3. Identifier and Target of Inhibitors Tested**

| **Inhibitor Identifier** | **Target** |
|---|---|
| SB239063 | MAP kinase |
| PD98059 | MAPKK |
| GSK-3b | GSK-3b |
| JNK | JNK |
| rapamycin | mTOR |
| everolimus | mTOR |
| Jak inhibitor 1 | Jak |
| CsA (cyclosporin A) | calcineurin |
| Tacrolimus | calcineurin |
| PD 153035 | EGFR |
| E804 | src, cdk/cyclin |
| Jak1 | Jak1 |
| Jak2 | Jak2 |
| Ly (=Ly294002) | PI3 kinase |
| PP2 | p561ck |
| SHP | SHP1/2 PTPase |
| STAT3p (STAT3 inhibitor peptide) | STAT3 |
| STAT3w (STAT3 inhibitor III, WP1066) | STAT3 |
| STAT3w (STAT3 inhibitor V) | STAT3 |
| STAT5 | STAT5 |
| AG490 | EGFR |
| U0126 | MEK1 |

Beta-actin expression served as a control for the expression assay. Offensive immune markers characterized included tumor necrosis factor superfamily (TNFSF) 1 (lymphotoxin), 2 (TNFα), 5 (CD40L), 6 (FasL), 8 (CD30L), 9 (CD137L), 14 (LIGHT), and 15 (TL1A). Chemokines characterized included CCL2, CCL3, CCL4, CCL8, CCL11, CCL20, CXCL1, CXCL2, CXCL3, CXCL10. Interleukins characterized include IL6, IL8, IL10, IL17, and IL23. Immune effectors characterized include GM-CSF, INFγ, CD16, Granzyme B, CD122, and TGFB-1. Markers of defensive immune function characterized include FoxP3, CTLA4, CD25, and GARP-1. Other offensive or defensive immune markers, as well as other chemokines, interleukins or effectors (among other immune-associated markers) can be tested in certain embodiments.

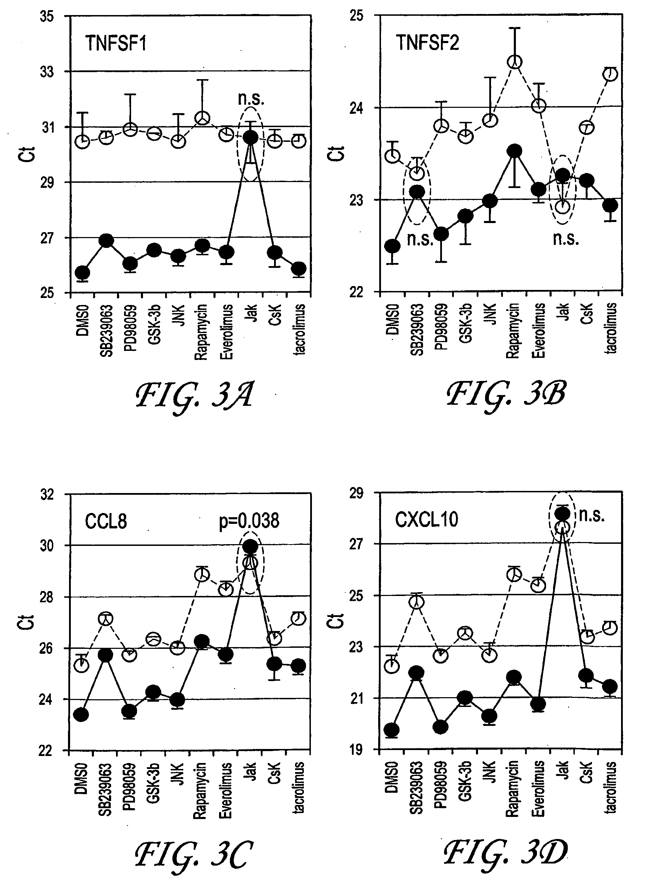

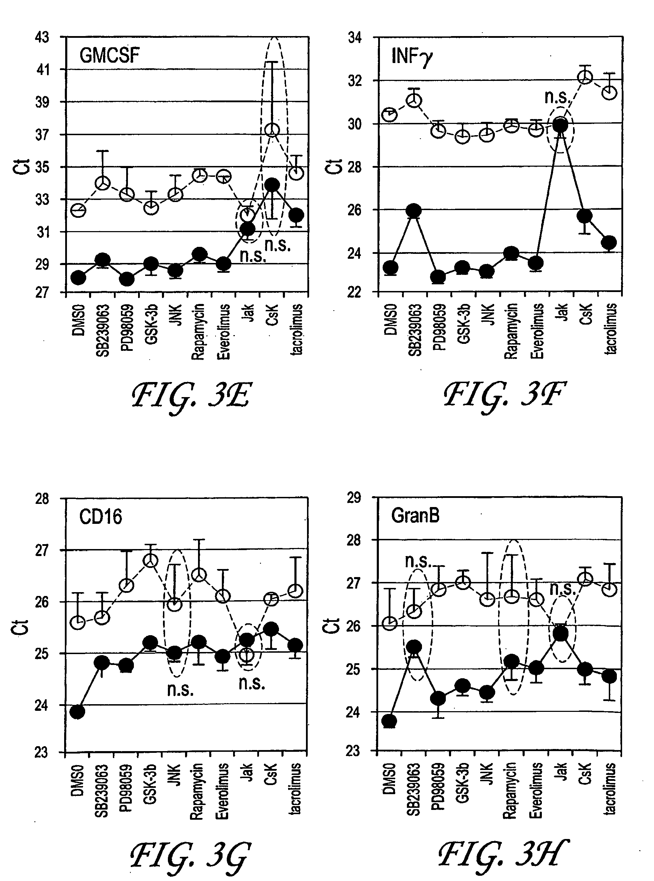

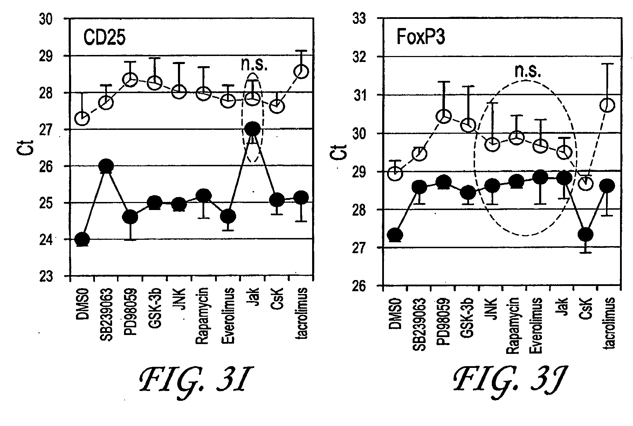

mRNA expression data of offensive and defensive immune markers is show in after rIl-2 stimulation is shown in Table 4. Shaded boxes represent a fold increase of greater than 2 as well as a statistical p value of <0.05. Shown in Figures 3A-3J are the expression profiles of selected offensive and defensive markers of immune function and their mRNA expression response to stimulation by PBS (open circles) or 100 ng/mL rIL-2 (closed circles). The various inhibitor compounds used to pre-treat the whole blood samples are listing on the x-axis. As can be seen from this data, the induction of certain offensive or defensive immune markers in the presence of an inhibitor suggest the potential of that compound to promote either offensive or defensive immunity. For example, as shown in Figure 3A, PBS-stimulation of blood samples and the measurement of induction of TNFSF-1 resulted in no significant expression changes (open circles). However, in the presence of rIL-2 as the stimulant, most blood samples show induction of TNFSF1 mRNA (offensive immune marker), except for the samples treated with an inhibitor of Janus kinase (Jak). This sample showed no induction of TNFSF1 in response to rIL-2. Thus, this compound has a potential to block or reduce the offensive immune response and may function as a pro-immunosuppressant compound. However, when evaluating the effect of the same compound on two markers of the defensive immune response, CD25 or FoxP3 (Figures 3I and 3J, respectively), the data indicate that rIL-2 blocks induction of these defensive markers. Thus, from this set of experiments, it is unclear if this particular Jak inhibitor is well-suited for promoting offensive or defensive immune responses. Further dose-response studies and/or studies with additional immune markers are necessary to elucidate the efficacy of this compound.

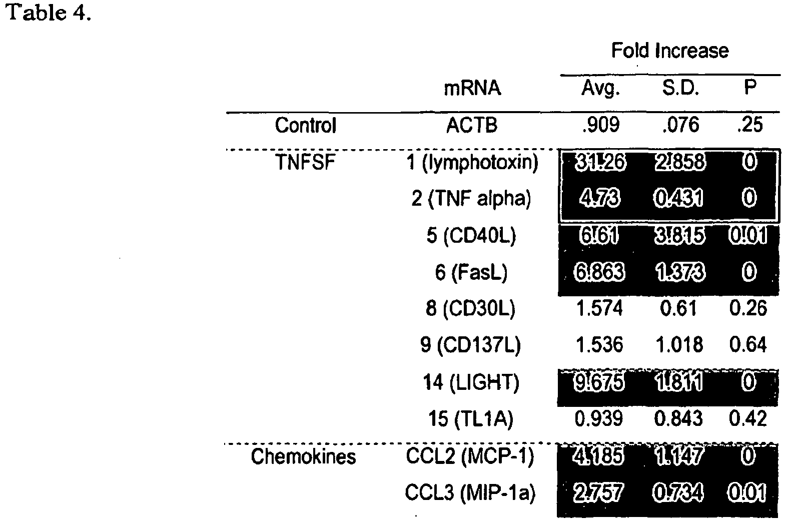

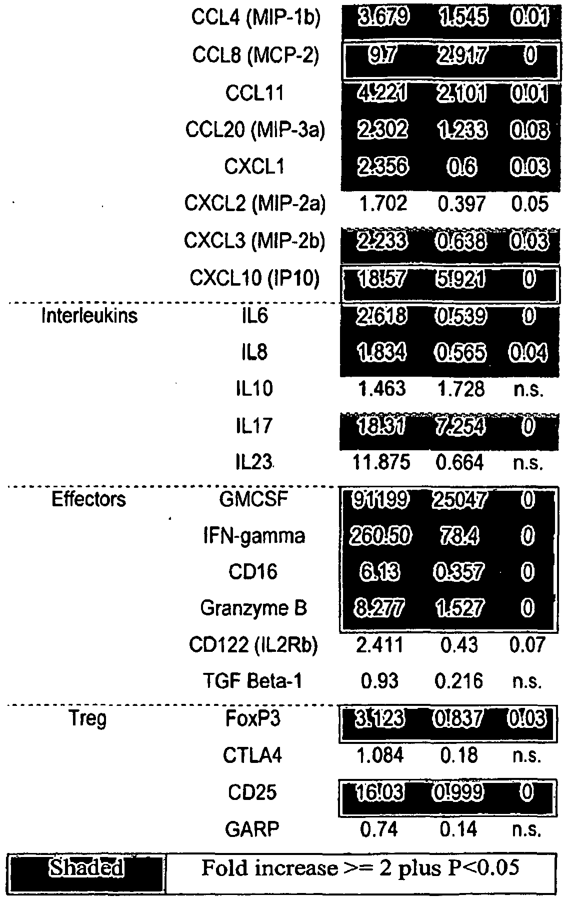

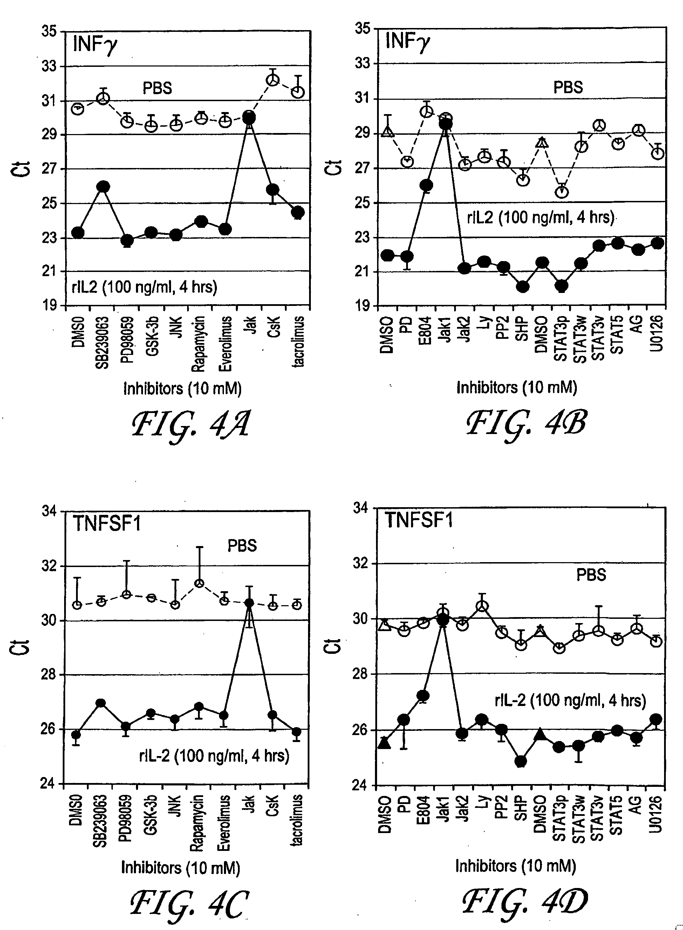

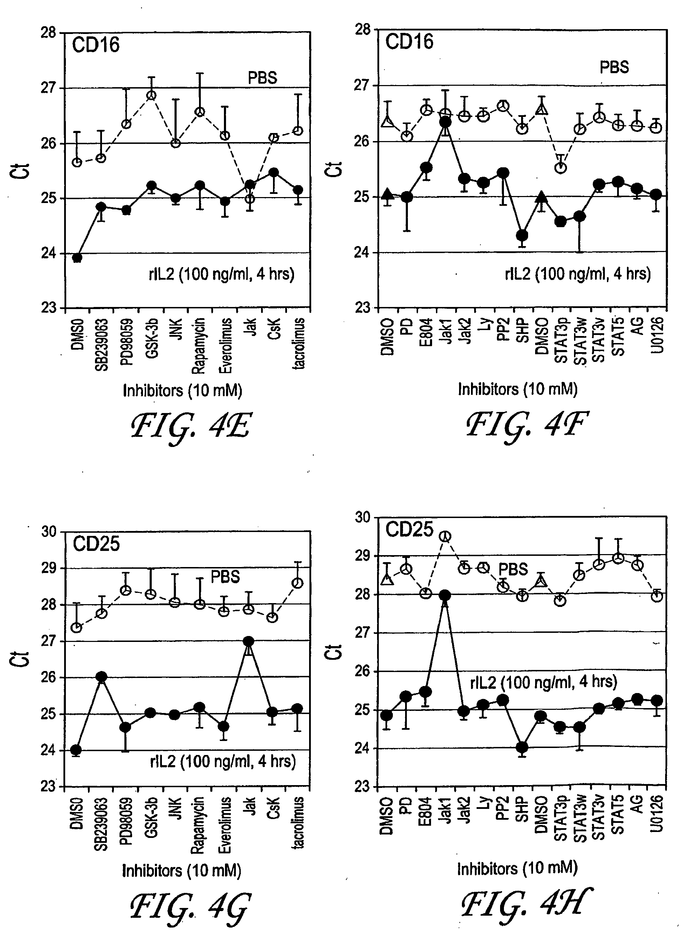

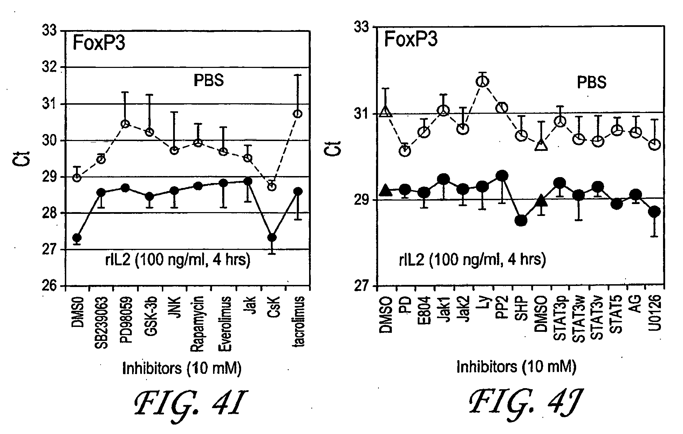

Figures 4A-4J depicts additional experiments measuring the induction of IFNγ (Figure 4A-4B), TNFSF1 (Figure 4C-4D), CD16 (Figure 4E-4F), CD25 (Figure 4G-4H), or FoxP3 (Figure 4I-4J) in response to PBS or rIL-2 stimulation in the presence or absence of various inhibitor compounds. These data indicate that Jak1 is a specific inhibitor of offensive immune markers (i.e. pro-defensive) as shown by the lack of induction of the offensive markers IFNγ and TNFSF-1 and the induction of expression of FoxP3, a defensive associated marker. Compound E084 also demonstrated a specific inhibition of the offensive markers IFNγ and TNFSF-1. However, E084 induced both CD25 and FoxP3, suggesting that E084 may be a more potent promoter of the defensive immune response. Embodiments of the methods described herein are also well suited to the characterization of various derivative or modified compounds, in that a single assay can generate side by side data comparing of a panel of putative derivative compounds. Moreover, based on the distinct features of the data described above, embodiments of the methods described herein are useful to detecting defensive-specific (i.e. pro-offensive) inhibitor compounds for use in clinical situations. Thus, this assay platform is useful for the analysis/screening of common and selective inhibitors for offensive and defensive immune functions.

### Example 3- Use of Offensive and Defensive Immune Marker mRNA Induction to Identify Tailored Therapies

Mature leukocytes circulating in peripheral blood are generally in a steady state, and once they migrate to local lesions of inflammation, neoplasms, foreign bodies (microorganisms, transplanted tissues and devices, drugs and vaccines), etc., they are fully activated in a specific manner. The specificity of activation is dependent on, among other factors, the type of leukocyte recruited and the type of local stimulants. In order to simulate *in vivo* leukocyte responses in an *in vitro* system, the present example exposed crude whole blood to various specific and general stimulants and the variety of leukocyte responses were quantified. The various leukocyte response were categorized based on their association with a particular type of immune response (e.g., humoral immunity, cell-mediated immunity, etc.)

In most in vitro assays, leukocytes are isolated and cultured with or without specific stimuli for a period of time (e.g., several days to several weeks) to identify functional changes in the leukocytes (e.g., protein synthesis and secretion, apoptosis, cell proliferation, surface marker changes, etc.). However, the complexity, cost, and duration of such assays severely limit their applicability as routine diagnostic tests. Several protocols were developed to attempt to overcome technical difficulties associated with cell isolation and culture conditions, such as, for example use of whole blood with short incubation (typically overnight) with specific stimuli, followed by quantification of adenosine-5'-triphosphate (ATP) levels or measurement of the levels of various cytokines by enzyme-linked immunosorbent assay (ELISA). However, the utility of knowing ATP levels is limited, as a wide variety of leukocyte functions are not ATP-dependent. Further, the detection limit of a typical ELISA is picomole to femtomole (10¹¹ to 10¹⁵ molecules), thus limiting the sensitivity of such protocols.

In contrast, methods according to several embodiments disclosed herein measure the *ex vivo* induction of leukocyte-function-associated mRNAs. Measuring mRNA levels is advantageous because polymerase chain reaction is capable of sensitivity down to single molecule detection and because mRNA induction happens much earlier than protein synthesis and corresponding biological changes (as would be measured by ELISA). As discussed herein, the use of whole blood maintains the *in vivo* complex cell-to-cell communication and interaction of leukocytes with plasma factors and proteins. While protocols exist for mRNA analysis in whole blood, such methods provide only a snapshot of gene expression at the time of blood draw. In contrast, embodiments disclosed herein analyze fluctuation in the levels of mRNA after appropriate stimulation, which provides a dynamic series of data points related to the leukocyte responses to stimuli.

Although PCR is sensitive enough to detect a single copy of target gene, several embodiments disclosed herein are particularly sensitive based on the reduced variation among triplicate aliquots of whole blood. In standard assays, variation can be induced at any step from leukocyte isolation, RNA purification, cDNA synthesis, to PCR. Because of the amplification-based nature of PCR, even minute variations introduced before PCR will be exponentially increased. Moreover, even from a single blood sample, many aliquots are generated based on the number of stimulants, dose responses, time course, combinations of stimulants, duplicate or triplicate, etc. Based on such limitations of the standard protocols known in the art, Applicant developed a high throughput assay platform, which is exploited in several embodiments disclosed herein. Further information regarding the assay platform may be found in U.S. Patent Applications Nos.: 10/796,298, 11/525,515, 11/376,018, 11/803,593, 11/803,594, and 11/803,663.

### Materials and methods

### Materials

Anti-T cell receptor α/β chain (TCR) monoclonal antibody (IgG1_{K}) and control mouse IgG1_{K} were obtained from BioLegend (San Diego, CA, USA). Reverse transcriptase, dNTP, and RNasin were purchased from Invitrogen (Carlsbad, CA, USA). All other chemicals were purchased from Sigma-Aldrich (St. Louis, MO, USA). Immune complex (heat aggregated IgG, HAG) was prepared by heating human IgG at 63°C for 15 min as described previously (Ostreiko et al., 1987). In 8-well strip microtubes, 1.2 µl each of phytohemagglutinin-L (2 mg/ml), HAG (10 mg/ml), lipopolysaccharide (LPS) (0.5 mg/ml), zymosan A (75 mg/ml), recombinant interleukin 2 (rIL2) (5 µg/ml), phosphate buffered saline (PBS), anti-TCR antibody (50 µg/ml), and control IgG (50 µg/ml), were added respectively into 8 wells, and stored at -80°C until use.

### Blood treatment

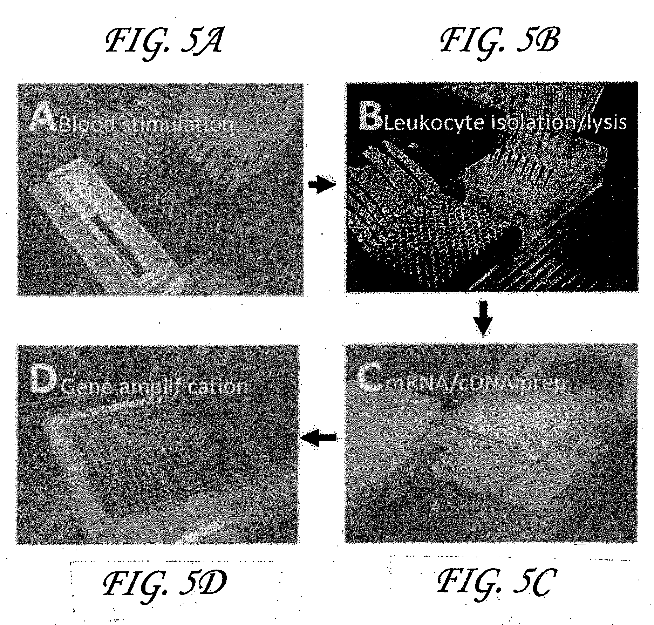

Heparinized whole blood samples were obtained from Apex Research Institute (Tustin, CA, USA) after Institutional Review Board approvals. In order to equalize post-blood collection condition, blood samples were stored at 4°C overnight. Next morning, blood was decanted into a reservoir and using an 8-well multi-channel pipette, 60 µl each of blood was dispensed into 3 strips containing the control agents or stimulants described above (Figure 5A). The blood volume needed for this test was 1.44 ml (60 µl/well x 8 wells x 3 strips (triplicate)). After cap was closed, strips were incubated at 37°C for 4 hours, then stored frozen at -80°C. It shall be appreciated that, in some embodiments, larger or smaller volumes of blood may be used (e.g., increased due to testing a larger number of stimulants or leukocyte-activation related genes). Two categories of patient were tested in this example, a normal healthy (control) subject (data in Table 5) and a patient having cancer (data in Table 6).

### Target mRNAs

The mRNA sequences of target mRNAs were retrieved from GenBank. PCR primers were designed within the coding region by Primer Express (Applied Biosystems (ABI), Foster City, CA, USA) (See Table 1). Oligonucleotides were synthesized by IDT (Coralville, IA, USA). The target mRNAs (total 32) were β-actin (ACTB), β2-microglobulin (B2M), granzyme B (GZB), perforin 1 (PRF1), tumor necrosis factor superfamily (TNFSF)-1, 2, 5, 14, and 15, CCL chemokines-2, 4, 8, and 20, CXCL chemokines-3, and 10, interleukin (IL)-2, 4, 6, 8, 10, and 17A, interferon-γ (IFNG), granulocyte-macrophage colony-stimulating factor (GMCSF), CD11a, 16 and 25, transforming growth factor beta 1 (TGFB1), forkhead box P3 (FOXP3), immunoglobulin heavy locus (IGH@), arginase (ARG), vascular endothelial growth factor (VEGF), and pro-opiomelanocortin (POMC).

### mRNA analysis

Fifty microliters of stimulated and frozen whole blood was thawed and applied to 96-well custom filterplates (Figure 5B). Leukocytes were isolated on the filter membranes by centrifugation. In some embodiments, other techniques may be used to isolate the leukocytes on the filter membrane (e.g., vacuum, positive pressure, gravity and the like). Further information on isolation of leukocytes may be found in U.S. Patent Application Nos.: 10/796,298, 11/525,515, 11/376,018, 11/803,593, 11/803,594, and 11/803,663. Sixty µL of lysis buffer containing a cocktail of specific reverse primers was applied to the filterplates, and the resultant cell lysates were transferred to oligo(dT)-immobilized microplates for poly(A)+ mRNA purification (Figure 5C). The cDNA was directly synthesized in 50 µL solutions at each well: specific primer-primed cDNA in the liquid phase and oligo(dT)-primed cDNA in the solid phase. The liquid and solid phase cDNAs were used for real time PCR using iTaqSYBR (Biorad, Hercules, CA, USA) in thermal cyclers (PRISM 7900, ABI, and iCycler, Biorad, respectively). PCR conditions were 95°C for 10 min followed by 50 cycles of 65°C for 1 min and 95°C for 30 sec. For IL2 and IL4, 4 µl of undiluted cDNA solution was used in the final volume of 10 µL in 384 well PCR plate. (Figure 5D). For FOXP3, ARG, IFNG, GMCSF, and POMC, 2 µl of undiluted cDNA solution was used for PCR in the final volume of 5 µL. The cDNA was then diluted 1:2 by adding 32 µL of DNase/RNase free water, and 2 µl each of cDNA was used for PCR for remaining 24 genes (except IL17A) in the final volume of 5 µL. After the leftover cDNA was transferred to fresh strip microtubes, solid phase cDNA was used directly to amplify IL17A by adding 30 µL PCR solution. The melting curve was always analyzed to confirm that PCR signals were derived from a single PCR product. The cycle threshold (Ct) was determined by analytical software (SDS, ABI), and statistical p values were calculated by t-test using 3 Ct values each of stimulant and control. The Ct of drug-treated triplicate samples were subtracted individually by the mean Ct values of control samples to calculate ΔCt, and the fold increase was calculated as 2^(-ΔCt).

### Results and Discussion

In the present example, 6 different stimulants (with 2 controls) were used (Tables 5 and 6, x-axis). PHA is a lectin commonly used to stimulate the general T-cell population. HAG is a model of immune complex to stimulate IgG Fc receptor (FcγR)-positive leukocytes. Unlike antibody-dependent cell-mediated cytotoxicity (ADCC), where a primary cellular target is CD16+ natural killer (NK) cells, stimulation with HAG targets CD16+, CD32+, and CD64+ cells. LPS and zymosan are commonly used agents to stimulate toll-like receptor (TLR) for the analysis of innate immunity. Since the anti-TCR antibody binds to the antigen recognition molecule of the T-cell receptor (α/β chain) of both CD4+ and CD8+ T-cells, it is used as a universal TCR antigen (Mitsuhashi et al., 2008c). Recombinant IL-2 binds to IL2-receptor positive T-cells, including regulatory T-cells (Treg), and induces a wide variety of immunological reactions.

Although immunity is extremely complex with numerous cellular and humoral factors interacting by each other, for purposes of predicting an effective individualized therapy as in several embodiments disclosed herein, such complexity can be simplified down to several functional categories as shown on in Tables 5 and 6.

Generally, in order for leukocytes to relocate from blood to local lesions, they first express CD11a on the cell surface, which binds to intercellular adhesion molecules expressed on endothelial cells damaged by a lesion. As shown in Tables 5 and 6, detection of increased CD11a expression was detected by stimulation of whole blood samples with zymosan. Once CD11 a-expressing leukocytes encounter target cells or molecules (e.g., those of the lesion), appropriate subsets of leukocytes must be recruited to the lesion. Expression of a variety of chemotactic CCL and CXCL chemokines were analyzed. CCL2 (monocytes and basophils), CCL4 (granulocytes), CCL8 (mast cells, eosinophils, basophils, monocytes, T cells, and NK cells), CCL20 (lymphocytes), CXCL3 (monocytes), CXCL8 (=IL8) (neutrophils), and CXCL10 (monocytes, macrophages, T cells, NK cells, and dendritic cells) are known as chemoattractants for respectively listed cell types. As shown in Tables 5 and 6, each stimulant induced different subsets of chemokines, which indicates a possible means to exploit (e.g., recruit) particular cell types based on an individual's chemotactic and chemokine expression profile. In some embodiments, an individual's chemotactic and chemokine expression profile provides data that enables a prediction of that individual's ability to develop cellular infiltration (e.g., deliver the appropriate immune cells to the target site) at disease sites. Such data is useful, in some embodiments, to predict the efficacy of and develop a tailored therapy. For example, if stimulation of whole blood of a cancer patient fails to induce expression of any chemotactic or chemokine molecules, that individual may be deficient in leukocyte recruiter function, and therefore would not be an ideal candidate for an immune based therapy (e.g., cancer immunotherapy). Rather, that individual is likely to respond better to a more traditional radiation, pharmacological, or surgical approach.

Once recruited to the site of a lesion, locally infiltrated leukocytes also induce a cascade of events designed to kill the target cells. Multiple mechanisms for killing a target cell can be employed, including induction of apoptosis. As shown in Tables 5 and 6, induction of Granzyme and perforin mRNA (well known inducers of apoptosis) by various stimulants were identified. Increased expression of CD 16, a specific marker of NK cells was also identified. The Tumor Necrosis Factor (ligand) Super Family (TNFSF) comprises a variety of inducers of apoptosis against target cells that are TNFSF receptor-positive. As shown in Tables 5 and 6, each stimulant induced different members of TNFSF. Thus, the data related to the offensive immune function of an individual may be used, in some embodiments, to characterize the potential of that patient to generate innate anti-cancer activity. Also, in some embodiments, the severity of autoimmune diseases can be characterized. Advantageously, the methods presented herein allow a characterization of the potential for a patient to respond to a particular type of therapy prior to administering the therapy. This is particularly advantageous in situations where a patient's survival is dependent on initiating an effective therapy as early as possible. As such, the knowledge that a cancer patient exhibits little inducement of expression of "killer" molecules suggests that such a patient would likely not benefit from such therapies and that other, non-immune, therapies should be investigated.

As discussed above, human immunity has a variety of negative regulators (also referred to herein as "defensive immune markers"), which include humoral (IL10 and TGFB1), and cellular components (Treg and myeloid derived suppressor cells (MDSC)). These regulators have the capacity to suppress or reduce the efficacy of an offensive (e.g., killer) immune response when induced. In contrast, their reduced expression may favor generation of auto-immune disorders. Thus, the balance of expression of negative regulators and recruiter/killer molecules are used, in some embodiments, to further assess the potential efficacy of a given type of therapy. As shown in Table 5, IL10 mRNA was induced by the anti-TCR antibody and zymosan, and TGFB1 mRNA was induced by zymosan. FOXP3 and CD25 mRNA were measured as the markers of Treg activity. While no stimulant induced FoxP3, CD25 was induced by all stimulants except HAG (see Table 5). Arginase mRNA was measured as a marker of MSDC function, though no induction was detected in Table 5.

As shown in Table 6, a patient with cancer appeared to have a greater degree of suppressor induction in response to the various stimuli. Zymosan significantly induced all suppressors analyzed except for TGF-beta1. Similarly, PHA induced 3 of the 5 suppressor markers analyzed. Thus in some embodiments, such data may suggest the origins of an illness, for example the enhanced expression of suppressors may have contributed to a reduced efficacy of killer (e.g., offensive) function, thereby facilitating the generation of a malignancy. Likewise, enhanced suppressor function, particularly in the absence of enhanced killer function, suggests that immune therapies would be ineffective for such an individual. µ

As the markers of various subsets of T helper cells, which primarily function to activate and direct other types of immune cells, the induction of IL2 (Th1), IFNG (Th1), IL4 (Th2), IL10 (Th2), and IL17 (Th17) mRNAs were identified (Tables 5 and 6). Induction of these markers suggest that there is capacity for multiple levels of the immune system are upregulated simultaneously, rather than simply an induction of offensive (e.g., killer) function cells. As such, in some embodiments, analysis of each of the various functional categories discussed above, taken together, are used to determine which type of treatment modality is likely to be effective. For example, upregulation of offensive markers in conjunction with a lack of induction of helper or recruitment markers suggests that the offensive cells, while having greater activity, may not be sufficiently well targeted or supported by other portions of the immune system. In such a case, immune-based therapies may not be ideal. In other embodiments, however, the magnitude of induction of a single category (or single marker) may be sufficient to merit an associated therapeutic regimen.

A variety of other functions are carried out in the immune system, such as the ability of the immune system to "learn" the identity of various foreign molecules, which is of critical importance in the humoral (e.g., antibody mediated) immune response. Granulocyte macrophage colony stimulating factor (GM-CSF) is functions as a white blood cell growth factor, and GMCSF mRNA was modestly induced by various stimulations in a healthy subject (Table 5). Likewise, GMCSF was upregulated in a cancer patient, though to a vastly greater degree than the healthy subject (Table 6). These data suggest that a cancer patient may have the capacity to make a large number of leukocytes, but the total number of cells may not guarantee effective immune function, but rather the eventual balance of the offensive and defensive functions may be a significant determining factor. As a marker of B-cells, which function to make antibodies against antigens and develop into memory B-cells, mRNA of IgG heavy chain (IGH@) was induced by zymosan in a healthy patient (Table 5), but not in a cancer patient (Table 6). Many lesions, such as cancers require an increased blood supply due to the higher rate of cellular metabolism and tissue growth. VEGF expression was studied as a marker of angiogenesis and was induced by zymosan in both the healthy and cancer patient. Although pain-associated POMC (endorphin) mRNA was not induced in these subjects, POMC mRNA has been shown to be induced by zymosan in other healthy control subjects in related experiments (data not shown).

As demonstrated in this example, the high-throughput methods were sensitive enough to characterize a wide spectrum of leukocyte function in a healthy individual and in an individual (Table 5) with cancer (Table 6). Using triplicate aliquots of whole blood for both stimulant and solvent controls, statistical conclusion were able to be drawn for each stimulant for each mRNA. While control genes such as ACTB and B2M were not induced (e.g., fold change >2) in the control individual, B2M was induced in the cancerous individual. However, since B2M is a component of the major histocompatibility complex, it is not unreasonable to consider that alterations in expression would be induced by stimulation of whole blood with zymosan. Regardless, control genes were not used to normalize the PCR results of other mRNAs. In fact, given the sensitivity of the methods used herein, statistical significance was often identified even fold increase was <2, or >0.6 (e.g.., rIL2- and PHA-induced ACTB, PHA-induced CCL4, zymosan-induced CXCL10, HAG- and zymosan-induced FOXP3 in Table 5). In contrast, some low copy number genes (e.g.., IL2, IL4, GMCSF, and POMC) exhibit large variation, and occasionally >10 fold increase was not significant (TCR-induced GMCSF in Table 5). Thus in Tables 5 and 6, >2 fold increase plus p<0.05 was considered as positive results (e.g., significant induction; dark background). The degree of induction of the various markers may not necessarily be directly linked to the degree of biological significance, for example, a large fold increase in expression of a low copy number gene may have less biological impact as compared to a small fold increase of an abundant gene. Thus, while the degree of change in expression may not necessarily be indicative of the degree of change in a particular pathway, these data are of particular value for determining the patterns of expression. As disclosed herein, the pattern of expression, in several embodiments, is used to make develop an individualize therapy or diagnosis based on the expression of certain categories of leukocyte-function associated markers. With the inclusion of general immune function stimulators such as rIL2, data generated by the methods disclosed herein are applicable to oncology and autoimmune diseases, among other disease types. According to several embodiments, several embodiments use the data generated in relation to developing therapies in the context of preclinical studies, clinical trials, as well as the development of companion diagnostics.

## Claims

1. A method for determining whether an immune-based therapy or a non-immune-based therapy is effective for a cancer patient, wherein the non-immune-based therapy is selected from the group consisting of a radiation, surgery or chemotherapy, said method comprising:
Exposing a first aliquot of whole blood from a cancer patient to a solvent comprising an immune stimulating agent selected from zymosan and
recombinant interleukin-2 (rIL2);
exposing a second aliquot to the solvent not comprising the immune stimulating agent;
quantifying the ratio between the amount of mRNA encoding an immune function-related mRNA selected from CD16, FOXP3, and arginase in said first and second aliquot after the exposure, thereby quantifying induction of the immune function-related mRNA;
(a) wherein an induction of CD16 upon exposure to the immune stimulating agent is indicative for an immune based therapy being effective,
(b) wherein an induction of FOXP3 upon exposure to the immune stimulating agent is indicative for a non-immune based therapy being effective, or
(c) wherein an induction of arginase upon exposure to the immune stimulating agent is indicative for a non-immune based therapy being effective.

2. The method according to Claim 1, wherein said whole blood is stored at room temperature or refrigeration for less than 1 day.

3. The method according to Claim 1 or 2, wherein said exposure of said first and said second aliquots is for less than 24 hours.

4. The method according to Claim 3, wherein said exposure is for between 2 and 6 hours.

5. The method according to Claim 4, wherein said exposure is for about 4 hours.

6. The method according to any one of Claims 1 to 5, wherein said whole blood is heparinized.

7. The method according to any one of Claims 1 to 6, wherein the immune-based therapy comprises an anti-cancer immunotherapy regimen.

## Patentansprüche

1. Verfahren zur Bestimmung, ob eine immunbasierte Therapie oder eine nicht-immunbasierte Therapie bei einem Krebspatienten wirksam ist, wobei die nicht-immunbasierte Therapie ausgewählt ist aus der Gruppe bestehend aus Bestrahlung, Chirurgie oder Chemotherapie, wobei das Verfahren umfasst:
Inkontaktbringen eines ersten Aliquots Vollblut eines Krebspatienten mit einem Lösungsmittel, das ein immunstimulierendes Agens, ausgewählt aus Zymosan und rekombinantem Interleukin-2 (rIL-2), umfasst;
Inkontaktbringen eines zweiten Aliquots mit dem Lösungsmittel, das das immunstimulierende Agens nicht umfasst;
Quantifizierung des Verhältnisses zwischen der Menge an mRNA, die eine immunfunktionsabhängige mRNA ausgewählt aus CD16, FOXP3 und Arginase kodiert, in dem ersten und zweiten Aliquot nach dem Inkontaktbringen, wodurch die Induktion der immunfunktionsabhängigen mRNA quantifiziert wird;
(a) wobei eine Induktion von CD16 nach dem Inkontaktbringen mit dem immunstimulierenden Agens anzeigt, dass eine immunbasierte Therapie wirksam ist;
(b) wobei eine Induktion von FOXP3 nach dem Inkontaktbringen mit dem immunstimulierenden Agens anzeigt, dass eine nicht-immunbasierte Therapie wirksam ist; oder
(c) wobei eine Induktion von Arginase nach dem Inkontaktbringen mit dem immunstimulierenden Agens anzeigt, dass eine nicht-immunbasierte Therapie wirksam ist.

2. Verfahren nach Anspruch 1, wobei das Vollblut bei Raumtemperatur oder gekühlt weniger als einen Tag lang gelagert wird.

3. Verfahren nach Anspruch 1 oder 2, wobei das Inkontaktbringen des ersten und zweiten Aliquots weniger als 24 Stunden dauert.

4. Verfahren nach Anspruch 3, wobei das Inkontaktbringen zwischen 2 und 6 Stunden dauert.

5. Verfahren nach Anspruch 4, wobei das Inkontaktbringen etwa 4 Stunden dauert.

6. Verfahren nach einem der Ansprüche 1 bis 5, wobei das Vollblut heparinisiert ist.

7. Verfahren nach einem der Ansprüche 1 bis 6, wobei die immunbasierte Therapie ein Anti-Krebs-Immuntherapie-Behandlungsschema umfasst.

## Revendications

1. Méthode pour déterminer si un traitement immunothérapeutique ou un traitement non immunothérapeutique est efficace pour un patient cancéreux, où le traitement non immunothérapeutique est choisi dans le groupe constitué de radiations, de chirurgie ou de chimiothérapie, ladite méthode comprenant :
l'exposition d'une première aliquote de sang total provenant d'un patient cancéreux à un solvant comprenant un agent immunostimulant choisi parmi le zymosan et
l'interleukine 2 recombinante (rIL2) ;
l'exposition d'une seconde aliquote au solvant ne comprenant pas l'agent immunostimulant ;
la quantification du rapport entre la quantité d'ARNm codant pour un ARNm associé à une fonction immunitaire choisi parmi les CD16, FOXP3, et arginase dans lesdites première et seconde aliquotes après l'exposition, quantifiant de cette façon l'induction de l'ARNm associé à une fonction immunitaire ;
(a) où une induction de CD16 après exposition à l'agent immunostimulant indique qu'un traitement immunothérapeutique est efficace,
(b) où une induction de FOXP3 après exposition à l'agent immunostimulant indique qu'un traitement non immunothérapeutique est efficace, ou
(c) où une induction de l'arginase après exposition à l'agent immunostimulant indique qu'un traitement non immunothérapeutique est efficace.

2. Méthode selon la revendication 1, dans laquelle ledit sang total est stocké à température ambiante ou sous réfrigération pendant moins de 1 jour.

3. Méthode selon la revendication 1 ou 2, dans laquelle ladite exposition de ladite première et de ladite seconde aliquotes est pendant moins de 24 heures.

4. Méthode selon la revendication 3, dans laquelle ladite exposition est pendant entre 2 et 6 heures.

5. Méthode selon la revendication 4, dans laquelle ladite exposition est pendant environ 4 heures.

6. Méthode selon l'une quelconque des revendications 1 à 5, dans laquelle ledit sang total est hépariné.

7. Méthode selon l'une quelconque des revendications 1 à 6, dans laquelle le traitement immunothérapeutique comprend un régime d'immunothérapie anticancéreuse.
